(19) Europäisches Patentamt
European Patent Office
Office européen des brevets

(11) **EP 4 208 116 B1**

(12) **EUROPEAN PATENT SPECIFICATION**

(45) Date of publication and mention
of the grant of the patent:
**20.08.2025   Bulletin 2025/34**

(21) Application number: **21770259.6**

(22) Date of filing: **06.09.2021**

(51) International Patent Classification (IPC):
*A61B 34/10* (2016.01)    *A61F 2/24* (2006.01)

(52) Cooperative Patent Classification (CPC):
**A61B 34/10; A61F 2/2496;** A61B 2017/00716;
A61B 2034/104; A61B 2034/105; A61B 2034/108

(86) International application number:
**PCT/EP2021/074475**

(87) International publication number:
**WO 2022/049283 (10.03.2022 Gazette 2022/10)**

(54) **PLANNING OF A HEART VALVE IMPLANTATION VIA PATIENT-TAILORED HEMODYNAMICS ANALYSIS**

PLANUNG DES EINSATZES EINER HERZKLAPPE MITTELS AUF EINEN PATIENTEN ZUGESCHNITTENER HÄMODYNAMISCHER ANALYSE

PLANIFICATION DE L'IMPLANTATION D'UN VALVE CARDIAQUE PAR ANALYSE HÉMODYNAMIQUE ADAPTÉE AU PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2020  EP 20194661**

(43) Date of publication of application:
**12.07.2023   Bulletin 2023/28**

(73) Proprietor: **Hi-D Imaging AG**
**8406 Winterthur (CH)**

(72) Inventors:
• **GÜLAN, Utku**
**8152 Opfikon (CH)**
• **KARAKAS, Özge**
**8057 Zürich (CH)**

(74) Representative: **Vossius & Partner**
**Patentanwälte Rechtsanwälte mbB**
**Siebertstraße 3**
**81675 München (DE)**

(56) References cited:
**US-A1- 2015 112 659    US-A1- 2018 174 068**

• **RUDENICK PAULA A. ET AL: "An in vitro phantom study on the influence of tear size and configuration on the hemodynamics of the lumina in chronic type B aortic dissections", JOURNAL OF VASCULAR SURGERY, vol. 57, no. 2, 1 February 2013 (2013-02-01), AMSTERDAM, NL, pages 464 - 474.e5, XP055861620, ISSN: 0741-5214, DOI: 10.1016/j.jvs.2012.07.008**
• **LAING JUSTIN ET AL: "Patient-specific cardiac phantom for clinical training and preprocedure surgical planning", JOURNAL OF MEDICAL IMAGING, SOCIETY OF PHOTO-OPTICAL INSTRUMENTATION ENGINEERS, 1000 20TH ST. BELLINGHAM WA 98225-6705 USA, vol. 5, no. 2, 1 April 2018 (2018-04-01), pages 21222, XP060107122, ISSN: 2329-4302, [retrieved on 20180323], DOI: 10.1117/1.JMI.5.2.021222**
• **ASTUDILLO PATRICIO ET AL: "Research Article Enabling Automated Device Size Selection for Transcatheter Aortic Valve Implantation", 3 November 2019 (2019-11-03), XP055796161, Retrieved from the Internet <URL:https://www. ncbi.nlm.nih.gov/pmc/articles/PMC6875021/pdf/ JITC2019-3591314.pdf> [retrieved on 20210416]**

## Description

### Field of the invention

The present invention relates to a method for planning a heart valve implantation in a subject. The present disclosure further relates to a method for determining performance of an implanted heart valve. The present disclosure further relates to a method and a system for determining subject specific blood flow characteristics for at least a portion of the heart and/or aorta.

### Background of the invention

Cardiovascular diseases are the biggest threat to our population, being responsible for one-third of deaths globally. Among the most common cardiovascular diseases are valvular heart diseases, caused when at least one of the four heart valves loses its functioning. After the diagnosis, depending on the severity, the patients may receive an operation, where an artificial heart valve is implanted on top of the native valve. Different heart valve replacement operations are being used in the clinics, ranging from a more traditional open-heart surgery, to less invasive techniques such as transcatheter heart valve replacement operations where the artificial heart valve can be implanted through a catheter. In recent years, minimally invasive valve replacement techniques have become widely adopted in clinics worldwide.

Minimally invasive valve operations are applied to four heart valves, namely aortic, mitral, pulmonary, and tricuspid valves. The most common application is transcatheter aortic valve implantation (TAVI), which is a widely used heart valve replacement operation technique in the clinics worldwide since their first introduction in the market in 2002. In these operations, an artificial valve is inserted through the femoral artery via a catheter. In this way, the immediate risks of the operation are significantly reduced. Therefore, application of minimally invasive heart valves is becoming more popular over time and taking over the more traditional applications. Although the TAVI operation is advantageous over risky classical clinical procedures such as the open heart surgery because it allows percutaneous procedure for the patient, the efficacy and durability of the TAVs are still not optimal after the implantation as they are relatively new technology and have only been actively used in the clinics for <10 years. Nevertheless, the TAVI technology is adopted quickly worldwide, and is shifting the sector toward more micro-invasive processes over time. Therefore, TAVI is becoming a big breakthrough in the field of cardiac surgery technology with the advancements in the technology. Yet, the patient specific hemodynamic effects after a TAVI operation need further investigations. TAVI still has many problems due to their leaflets, implantation conditions, anatomy differences, hemodynamical effects and structural valve degeneration, leading to a significant number of revision operations and rehospitalization. In particular, the design of the valves, implantation parameters, anatomical differences, and valve degeneration are still being improved in order to improve the patient outcome.

Studies show that the implantation of transcatheter valves can lead to a significant number of post-operational problems that can result in revision operations and rehospitalization. Approximately one-third of the patients show poor outcomes such as heart failure and one-fifth of patients post-TAVI are readmitted to the hospital, which is associated with poor patient outcomes. As a result, treatments such as pacemaker implantation or revision operations are conducted. Post-operational problems and re-admission after TAVI show increasing trends years after the TAVI operation. Although the immediate risks are reduced, studies show that the long-term effects of TAVI are still not optimal. Early readmission of the patient is mainly due to complications related to the procedure, such as bleeding, renal failure. On the other hand, late readmission is mainly determined by baseline comorbidities, particularly among the elderly population, including a frailty criterion. In order to reduce hospital readmissions, reoperation rates and improve patient outcomes post-TAVI operations, considerable measures should be taken.

Hemodynamics concerns about the dynamics of the blood flow in the circulatory system. The human heart is a well-functioning pump, which circulates the blood in the circulatory system by pumping the blood in and out of the heart. The main duty of the human heart is to pump oxygenated blood to the cells via the circulatory system in the entire human body and then collect or pump deoxygenated blood back to the heart. The deoxygenated blood is then pumped to the lungs, where those blood cells pick up oxygen. This process repeats over the lifetime of the human life. Like every mechanical pump, the human heart starts losing its functioning over time and instabilities in hemodynamic conditions occur. This results in turbulent (chaotic) conditions in the blood flow, resulting in energy losses which influences the cardiac efficiency. During the blood flow in the circulatory system, it is important that the blood always flows in a certain direction with a relatively laminar (less chaotic) flow conditions, with minimal energy losses, turbulence in the flow, or backflow, otherwise active cells would not get the oxygen that is needed by the body or be able to release carbon dioxide when needed. Hence, it is important to maintain optimal blood flow conditions, especially after heart valve replacement operations where the native valve is replaced. A sub-optimal selection or implantation of the artificial valve can result in post-operational problems including reoperation and readmission. The hemodynamics analysis is crucial to help design an optimal new generation artificial valve, optimize the cardiac operational conditions for each patient's specific anatomy, maintain healthy and laminar blood flow conditions for the patient, improve post-operational conditions and patients' quality of lives and design a new generation artificial valve.

The aim of the invention is to improve the clinical decision-making workflow and to build a bridge between the heart teams at the clinics and heart valve manufacturers to provide the best treatment for patients with cardiovascular diseases and (1) provide patient-tailored therapy; (2) increase success rate of the transcatheter aortic valve replacement; (3) increase patient's quality of life; and (4) optimize the design of the next generation heart valves.

WO2015017571A1 discloses certain system for determining cardiovascular information for a patient, certain method for determining cardiovascular information for a patient using at least one computer, and certain non-transitory computer readable medium for use on at least one computer system containing computer-executable programming instructions for performing certain method for determining cardiovascular information for a patient.

US 2018/174068 discloses certain method for providing a subject-specific computational model of at least one component in the cardiovascular system for simulating blood flow and/or structural features.

US 2015/0112659 discloses certain method for patient-specific virtual percutaneous implantation, certain system for virtual percutaneous implantation, and certain implant for virtual percutaneous implantation obtained according to the disclosed method.

Laing Justin et al. (Journal of Medical Imaging, Society of Photo-Optical Instrumentation Engineers, vol. 5, no. 2, 1 April 2018, page 21222, DOI: 10.1117/1.JMI.5.2.021222) disclose patient-specific cardiac phantom for clinical training and pre-procedure surgical planning.

Astudillo Patricio et al. (URL: https://www.ncbi.nlm.nih.gov/pmc/articles/PMC6875021 /pdf/JITC2019-3591314.pdf, retrieved on 2021.04.016) disclose certain aspects of enabling automated device size selection for transcatheter aortic valve implantation.

## Summary of the invention

It is an object of the present invention to provide means and methods to increase the success rate of aortic valve replacement. It is a further object of the present invention to provide means and methods to improve the outcomes of minimally invasive cardiac operations. It is again a further object of the present invention to provide means and methods for determining performance of an implanted heart valve. It is again a further object of the present invention to provide means and methods for determining subject-specific blood flow characteristics. The technical problem is solved by the embodiments described herein and as characterized in the claims.

Also discloded herein is a method for planning a heart valve implantation in a subject, the method comprising (a) providing a subject-specific three-dimensional representation of the geometry of at least a portion of the heart and/or aorta and of blood flow therein, (b) determining blood flow characteristics using a previously trained mathematical model in the presence and absence of an implanted heart valve based on the three-dimensional representation obtained in (a) and varied size, design, implantation depth, and/or orientation of the implanted heart valve, and (c) determining the size, design, implantation depth, deployment thickness and/or orientation of the implanted heart valve based on the determined blood flow characteristics in the presence and absence of the implanted heart valve, wherein the size, design, implantation depth, deployment thickness, and/or orientation of the implanted heart valve are determined to minimize pressure drop, regurgitation volume, turbulence intensity, high shear stress regions, stagnation regions and/or flow separation regions.

In a particular aspect, the method for planning a heart valve implantation in a subject of the present invention relates to an embodiment, wherein the blood flow characteristics comprise blood flow patterns, velocity, retrograde flow rate, kinetic energy, vorticity, helicity, turbulence intensity, energy loss and/or shear stress.

In a further particular aspect, the method for planning a heart valve implantation in a subject of the present invention relates to an embodiment, wherein the blood flow characteristics are temporally and/or spatially resolved.

In again a particular aspect, the method for planning a heart valve implantation in a subject of the present invention relates to an embodiment, wherein the blood flow characteristics are determined in systolic and/or diastolic phase.

In again a particular aspect, the method for planning a heart valve implantation in a subject of the present invention relates to an embodiment, wherein the previously trained mathematical model is a machine learning-based method, preferably a deep learning network.

In again a particular aspect, the method for planning a heart valve implantation in a subject of the present invention relates to an embodiment, wherein the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

In again a particular aspect, the method for planning a heart valve implantation in a subject of the present invention relates to an embodiment, wherein the three-dimensional representation of the geometry of the subject's heart and/or aorta is obtained from MRI, ECHO, and/or CT imaging, preferably wherein the MRI, ECHO and/or CT images are automatically quantified and segmented by using a machine learning-based method, preferably wherein the machine learning-based method comprises a computer vision method.

In a further particular aspect, the method for planning a heart valve implantation in a subject of the present invention relates to an embodiment, wherein the previously trained mathematical model relies on a training dataset comprising experimental blood flow characteristics in phantoms of at least the portion of the heart and/or aorta.

In again a further particular aspect, the method for planning a heart valve implantation in a subject of the present invention relates to an embodiment, wherein the training dataset comprises the blood flow characteristics obtained from one or more subject(s) and/or through simulation(s).

Further in a particular aspect, the method for planning a heart valve implantation in a subject of the present invention relates to an embodiment, wherein the phantom of at least the portion of the heart and/or aorta is a three-dimensional model, preferably obtained using additive manufacturing techniques according to the geometry of the heart and/or aorta of a subject, preferably using elastic and/or transparent material.

In again a particular aspect, the method for planning a heart valve implantation in a subject of the present invention relates to an embodiment, wherein the experimental blood flow characteristics are obtained using optical imaging techniques, preferably 2D/3D Particle Imaging and/or Particle Tracking Velocimetry.

Further in a particular aspect, the method for planning a heart valve implantation in a subject of the present invention relates to an embodiment, wherein the training dataset comprises the experimental blood flow characteristics in the phantoms of at least the portion of the heart and/or aorta, determined in the presence of pathological conditions and/or implanted medical devices.

Further in a particular aspect, the method for planning a heart valve implantation in a subject of the present invention relates to an embodiment, wherein the training dataset comprises the experimental blood flow characteristics in the phantoms of at least the portion of the heart and/or aorta, determined in the presence and absence of pathological conditions and/or implanted medical devices.

In a further aspect, the present invention relates to a method for determining performance of an implanted heart valve, the method comprising (a) providing a subject-specific three-dimensional representation of the geometry of at least a portion of the heart and/or aorta, (b) obtaining a subject-specific phantom of at least the portion of the heart and/or aorta, preferably using additive manufacturing techniques according to the three-dimensional representation obtained in (a), preferably using elastic and/or transparent material, more preferably elastic, compliant and/or transparent material, (c) using optical imaging techniques to obtain experimental blood flow characteristics in the subject-specific phantom of at least the portion of the heart and/or aorta in the presence and absence of the implanted heart valve, and (d) determining performance of the implanted heart valve based on the experimental blood flow characteristics obtained in (c), wherein determined performance of the implanted heart valve comprises pressure drop, regurgitation volume, turbulence intensity, high shear stress regions, stagnation regions and/or flow separation regions.

In a particular aspect, the method for determining performance of an implanted heart valve of the present invention relates to an embodiment, wherein the experimental blood flow characteristics comprise blood flow patterns, velocity, acceleration, retrograde flow rate, mean kinetic energy, turbulent kinetic energy, vorticity, helicity, turbulence intensity, energy loss, stroke work and/or shear stress.

In a particular aspect, the method for determining performance of an implanted heart valve of the present invention relates to an embodiment, wherein the experimental blood flow characteristics comprise blood flow patterns, velocity, retrograde flow rate, kinetic energy, vorticity, helicity, turbulence intensity, energy loss and/or shear stress.

In again a particular aspect, the method for determining performance of an implanted heart valve of the present invention relates to an embodiment, wherein the experimental blood flow characteristics are temporally and/or spatially resolved.

In a further particular aspect, the method for determining performance of an implanted heart valve of the present invention relates to an embodiment, wherein the experimental blood flow characteristics are determined in systolic and/or diastolic phase.

Further in a particular aspect, the method for determining performance of an implanted heart valve of the present invention relates to an embodiment, wherein the optical imaging techniques comprise 2D/3D Particle Imaging and/or Particle Tracking Velocimetry.

In again a particular aspect, the method for determining performance of an implanted heart valve of the present invention relates to an embodiment, wherein the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

In again a particular aspect, the method for determining performance of an implanted heart valve of the present invention relates to an embodiment, wherein the three-dimensional representation of the geometry of the subject's heart and/or aorta is obtained from MRI, ECHO and/or CT imaging, preferably wherein the MRI, ECHO and/or CT images are automatically quantified and segmented by using a machine learning-based method, preferably wherein the machine learning-based method comprises a computer vision method.

In a further aspect, the present invention relates to a method for determining subject-specific blood flow characteristics for at least a portion of the heart and/or aorta, the method comprising (a) providing a subject-specific three-dimensional representation of the geometry of at least the portion of the heart and/or aorta and of blood flow therein; and (b) determining the blood flow characteristics based on the three-dimensional representation obtained in (a) using a previously trained mathematical model.

In a particular aspect, the method for determining subject-specific blood flow characteristics for at least a portion of the heart and/or aorta of the present invention relates to an embodiment, wherein the blood flow characteristics comprise blood flow patterns, velocity, acceleration, retrograde flow rate, mean kinetic energy, turbulent kinetic energy, vorticity, helicity, turbulence intensity, energy loss, stroke work and/or shear stress.

In a particular aspect, the method for determining subject-specific blood flow characteristics for at least a portion of the heart and/or aorta of the present invention relates to an embodiment, wherein the blood flow characteristics comprise blood flow patterns, velocity, retrograde flow rate, kinetic energy, vorticity, helicity, turbulence intensity, energy loss and/or shear stress.

In a further particular aspect, the method for determining subject-specific blood flow characteristics for at least a portion of the heart and/or aorta of the present invention relates to an embodiment, wherein the blood flow characteristics are temporally and/or spatially resolved.

In again a particular aspect, the method for determining subject-specific blood flow characteristics for at least a portion of the heart and/or aorta of the present invention relates to an embodiment, wherein the blood flow characteristics are determined in systolic and/or diastolic phase.

Further in a particular aspect, the method for determining subject-specific blood flow characteristics for at least a portion of the heart and/or aorta of the present invention relates to an embodiment, wherein the previously trained mathematical model is a machine learning-based method, preferably a deep learning network.

In again a particular aspect, the method for determining subject-specific blood flow characteristics for at least a portion of the heart and/or aorta of the present invention relates to an embodiment, wherein the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

In a further particular aspect, the method for determining subject-specific blood flow characteristics for at least a portion of the heart and/or aorta of the present invention relates to an embodiment, wherein the three-dimensional representation of the geometry of the subject's heart and/or aorta is obtained from MRI, ECHO and/or CT imaging, preferably wherein the MRI, ECHO and/or CT images are automatically quantified and segmented by using a machine learning-based method, preferably wherein the machine learning-based method comprises a computer vision method.

In again a further particular aspect, the method for determining subject-specific blood flow characteristics for at least a portion of the heart and/or aorta of the present invention relates to an embodiment, wherein the previously trained mathematical model relies on a training dataset comprising experimental blood flow characteristics in phantoms of at least the portion of the heart and/or aorta.

Further, in a particular aspect, the method for determining subject-specific blood flow characteristics for at least a portion of the heart and/or aorta of the present invention relates to an embodiment, wherein the training dataset comprises blood flow characteristics obtained from one or more subject(s) and/or through simulation(s).

In a further particular aspect, the method for determining subject-specific blood flow characteristics for at least a portion of the heart and/or aorta of the present invention relates to an embodiment, wherein the phantom of at least the portion of the heart and/or aorta is a three-dimensional model, preferably obtained using additive manufacturing techniques according to the geometry of the heart and/or aorta of a subject, preferably using elastic and/or transparent material, more preferably using anatomically accurate, elastic, compliant, and/or transparent material.

In again a particular aspect, the method for determining subject-specific blood flow characteristics for at least a portion of the heart and/or aorta of the present invention relates to an embodiment, wherein the experimental blood flow characteristics are obtained by using optical imaging techniques, preferably 2D/3D Particle Imaging and/or Particle Tracking Velocimetry.

Further in again a further particular aspect, the method for determining subject-specific blood flow characteristics for at least a portion of the heart and/or aorta of the present invention relates to an embodiment, wherein the training dataset comprises the experimental blood flow characteristics in the phantoms of at least the portion of the heart and/or aorta, determined in the presence and absence of pathological conditions and/or implanted medical devices.

Further in again a further particular aspect, the method for determining subject-specific blood flow characteristics for at least a portion of the heart and/or aorta of the present invention relates to an embodiment, wherein the training dataset comprises the experimental blood flow characteristics in the phantoms of at least the portion of the heart and/or aorta, determined in the presence of pathological conditions and/or implanted medical devices.

In a further aspect, the present invention relates to a system for determining subject-specific blood flow characteristic, the system comprising at least one computer configured to (a) receive a subject-specific three-dimensional representation of the geometry of at least a portion of the heart and/or aorta, (b) optionally receive information about size, design, implantation depth, deployment thickness and/or orientation of an implanted heart valve, and (c) determine the blood flow characteristics and/or risk analysis based on the three-dimensional representation received in (a) and optionally on size, design, implantation depth, deployment thickness, and/or orientation of the implanted heart valve received in (b) by using a previously trained mathematical model.

In a further aspect, the present invention relates to a system for determining subject-specific blood flow characteristic, the system comprising at least one computer configured to (a) receive a subject-specific three-dimensional representation of the geometry of at least a portion of the heart and/or aorta, (b) optionally receive information about size, design, implantation depth, deployment thickness and/or orientation of an implanted heart valve, and (c) determine the blood flow characteristics based on the three-dimensional representation received in (a) and optionally on size, design, implantation depth, deployment thickness, and/or orientation of the implanted heart valve received in (b) by using a previously trained mathematical model.

In a particular aspect, the system for determining subject-specific blood flow characteristic of the present invention relates to an embodiment, wherein the blood flow characteristics comprise blood flow patterns, velocity, retrograde flow rate, kinetic energy, vorticity, helicity, turbulence intensity, energy loss and/or shear stress.

In again a particular aspect, the system for determining subject-specific blood flow characteristic of the present invention relates to an embodiment, wherein the blood flow characteristics are temporally and/or spatially resolved.

In a further particular aspect, the system for determining subject-specific blood flow characteristic of the present invention relates to an embodiment, wherein the blood flow characteristics are determined in systolic and/or diastolic phase.

In again a further particular aspect, the system for determining subject-specific blood flow characteristic of the present invention relates to an embodiment, wherein the previously trained mathematical model is a machine learning-based method, preferably a deep learning network, wherein preferably the deep learning network is implemented using GPU-based cloud computing.

In a further particular aspect, the system for determining subject-specific blood flow characteristic of the present invention relates to an embodiment, wherein the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

In a further particular aspect, the system for determining subject-specific blood flow characteristic of the present invention relates to an embodiment, wherein the three-dimensional representation of the geometry of the subject's heart and/or aorta is obtained from MRI, ECHO and/or CT imaging, preferably wherein the MRI, ECHO and/or CT images are automatically quantified and segmented by using a machine learning-based method, preferably wherein the machine learning-based method comprises a computer vision method.

In again a particular aspect, the system for determining subject-specific blood flow characteristic of the present invention relates to an embodiment, wherein the previously trained mathematical model relies on a training dataset comprising experimental blood flow characteristics in phantoms of at least the portion of the heart and/or aorta.

Further, in a particular aspect, the system for determining subject-specific blood flow characteristic of the present invention relates to an embodiment, wherein the training dataset further comprises the blood flow characteristics obtained from one or more subject(s) and/or through simulation(s).

Further, in again a particular aspect, the system for determining subject-specific blood flow characteristic of the present invention relates to an embodiment, wherein the phantom of at least the portion of the heart and/or aorta is a three-dimensional model, preferably obtained using additive manufacturing techniques according to the geometry of the heart and/or aorta of a subject, preferably using elasticand/or transparent material, more preferably using elastic, compliant, and/or transparent material.

In a further particular aspect, the system for determining subject-specific blood flow characteristic of the present invention relates to an embodiment, wherein the experimental blood flow characteristics in the phantoms of at least the portion of the heart and/or aorta are obtained by using optical imaging techniques, preferably 2D/3D Particle Imaging and/or Particle Tracking Velocimetry.

In again a particular aspect, the system for determining subject-specific blood flow characteristic of the present invention relates to an embodiment, wherein the training dataset comprises the experimental blood flow characteristics in the phantoms of at least the portion of the heart and/or aorta, determined in the presence and absence of pathological conditions and/or implanted medical devices.

In again a particular aspect, the system for determining subject-specific blood flow characteristic of the present invention relates to an embodiment, wherein the training dataset comprises the experimental blood flow characteristics in the phantoms of at least the portion of the heart and/or aorta, determined in the presence of pathological conditions and/or implanted medical devices.

## Detailed description of the invention

The methods of the present invention are described in the following. It is however envisaged that all possible combinations are also included.

In one embodiment, the present invention relates to a method for planning a heart valve implantation in a subject. A subject herein is preferably a human subject, although a skilled person can envisage generalization of the methods of the present invention to any mammalian subject, or any vertebrate subject.

The first step of the method comprises providing a subject-specific three-dimensional representation of the geometry of at least a portion of the heart and/or aorta and of blood flow therein. In certain embodiments of the present invention, the subject-specific three-dimensional representation of the geometry of at least a portion of the heart and/or aorta and subject-specific three-dimensional representation of the of blood flow in at least a portion of the heart and/or aorta are provided separately. In certain embodiments, the subject-specific three-dimensional representation of the geometry of at least a portion of the heart and/or aorta and of blood flow therein can be obtained from one dataset by using the methods of the present invention, for example by relying on 4D MRI data. In certain preferred embodiments, the three-dimensional representation of the blood flow in at least a portion of the heart and/or aorta can be obtained by using Particle Imaging Velocimetry and/or Particle Tracking Velocimetry, as described herein.

In certain embodiments of the present invention, in the first step of said method, a machine learning-based method is used to automatically quantify, segment and reconstruct 3D geometries of the circulatory system, in certain embodiments of at least a portion of the heart and/or aorta, from MRI and CT images, in certain embodiments from MRI, ECHO, and/or CT images, in certain embodiments from MRI and/or CT images. The present inventors have demonstrated that the methods of the present invention can quantify, reconstruct and segment said 3D geometries, as accurately as when performed by experienced physicians in the course of manual measurements.

In certain embodiments of the present invention, the said method for planning a heart valve implantation in a subject preferably predicates on the blood flow characteristics and/or involves determining the blood flow characteristics of the subject. Preferably, based on blood flow characteristics for different scenarios, the best way to implant the heart valve to optimize the blood flow parameters, is devised. As understood herein, optimizing the blood flow characteristics is known to the skilled person and may involve e.g. minimizing the shear stresses, minimizing the energy drop, and the like. In certain embodiments of the present invention, the planning of a heart valve transplant is preferably performed using blood flow analysis, i.e. analysis of the blood flow characteristics. Further preferably, in certain embodiments of the present invention, training of the previously trained mathematical model relies on experimental data, preferably on in-vitro measurements from which the blood flow characteristics are obtained.

In certain embodiments of the present invention, the previously trained mathematical model can utilize the predicted blood flow characteristics to assess the risk, for example by predicting high turbulent intensity, and/or high shear stress, among others. The regions of the three dimensional representation of the geometry of at least a portion of the heart and/or aorta that are characterized by high turbulent intensity, and/or high shear stress can be defined as the risk regions or risk zones. As understood herein, "high" as referring to high turbulent intensity and/or high shear stress preferably refers to values at least 40%, preferably at least 50% higher than in the neighbouring area. Preferably, the risk regions based on blood flow characteristics as defined herein can be mapped onto the subject-specific three-dimensional representation of the geometry of at least a portion of the heart and/or aorta, and/or onto the subject-specific three-dimensional representation of the of blood flow in at least a portion of the heart and/or aorta.

Herein, the term "image" of the dimensions M x N in 2D space, or of the dimensions M x N x D in 3D space, defines a function I(i,j) in 2D space or I(i, j, k) in 3D space, where i = 0, ..., M - 1, j = 1, ..., N - 1, and k = 0, ..., D - 1 denote spatial coordinates. The values of the functions I(i, j) and I(i, j, k) are intensity values and are typically represented by a gray value {0, .... 255}. Each image consists of a finite set of image elements called pixels in 2D space or voxels in 3D space. Each said image element is uniquely specified by its intensity value and its coordinates (i, j) for pixels and (i, j, k) for voxels, respectively, wherein i is the image row number, j is the image column number, and k is the slice number in a volumetric stack.

The images referred to herein may be images obtained in the magnetic resonance imaging, or the MRI, measurements. The images may however be also obtained in the course of computer tomography scan, or a CT scan. In certain embodiments, the images are obtained from ultrasound imaging, which preferably may also be referred to as ECHO. Any suitable imaging method can be used to generate images as referred to in the present invention.

The term "to quantify" when referring to 3D geometries, for example of the circulatory system, preferably originating from MRI and CT images, or any other suitable imaging technique, refers to measurement of the geometric features of said 3D geometries. It is known to the skilled person that appropriate scale has to be applied to the images, so that pixels, of the image can be described in the units of length, and to describing the geometric features of the images using the physical units of length. For example, in certain experimental setups, 1 pixel of an image can correspond to the length of 1 mm.

The term "to segment" refers to a step of image processing comprising image segmentation. The goal of image segmentation is to divide an image into a set of preferably homogeneous, and nonoverlapping regions of similar attributes such as intensity, depth, color, or texture. The segmentation result is either an image of labels identifying each homogeneous region or a set of contours which describe the region boundaries. Herein, segmentation of 3D geometries of the circulatory system from MRI and CT images results in defining boundaries of the elements of the circulatory system, including portion of the heart and/or aorta. Image segmentation can be performed on 2D images (preferably representative of different orientations), sequences of 2D images, and 3D reconstructed geometry. Image segmentation can also be performed on 2D images, sequences of static 2D images, or time dependent 2D images. In the methods of the present invention, preferably segmentation is performed on 2D images. Alternatively, in the methods of the present invention, preferably segmentation is performed on both 2D images and 3D reconstructed geometry As it is understood to the skilled person, the sequences of 2D images (i.e., static 2D images) can be used to generate a 3D reconstructed image, which may also be referred to as 3D reconstructed geometry.

The term "to reconstruct" refers to a process of image processing and turning the images into geometric information on certain anatomical features, herein 3D geometries of the circulatory system, preferably a subject-specific three-dimensional representation of the geometry of at least a portion of the heart and/or aorta.

In certain embodiments, the three-dimensional representation of the geometry of the subject's heart and/or aorta is obtained from MRI and/or CT imaging, preferably wherein the MRI and/or CT images are automatically quantified and segmented by using a machine learning-based method. Preferably, in certain embodiments, the three-dimensional representation of the geometry of the subject's heart and/or aorta is obtained from MRI, ECHO and/or CT imaging, preferably wherein the MRI, ECHO and/or CT images are automatically quantified and segmented by using a machine learning-based method. In certain preferred embodiments, the machine learning-based method comprises a computer vision method. In alternative certain preferred embodiments, the machine learning-based method is a deep learning method.

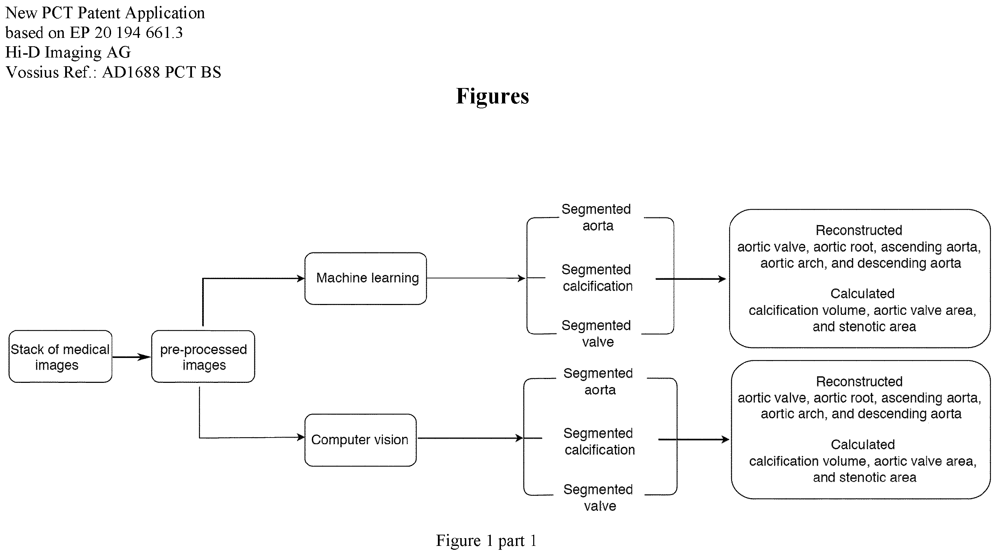

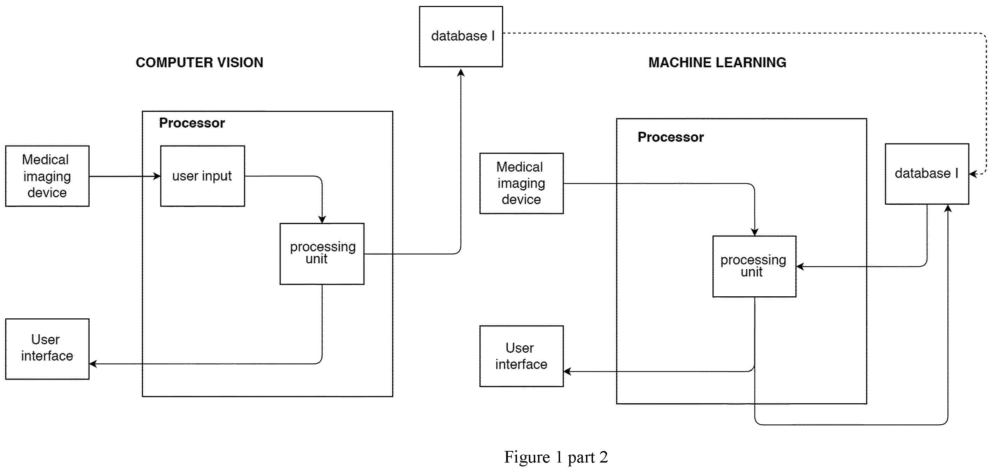

Figure 1 (upper panel) represents a schematic view of the method of the present invention that provides a subject-specific three-dimensional representation of the geometry of at least a portion of the heart and/or aorta. In certain embodiments of the present invention, a stack of images from MRI or CT serves as an input. In certain embodiments the stack of medical images is converted into a pre-processed volume, which is then segmented, preferably by using computer vision and/or machine learning. In certain embodiments, processing unit uses the previously trained mathematical model to find the segment the aorta and sends it to the database I (marked as Database in the lower panel of Figure 1). Preferably, segmentation of full aorta (root, ascending, arch, descending), calcified regions, and valve leaflets including the stenotic region is performed. In certain embodiments, the reconstructed 3D geometry of aorta, of calcification as well as stenosis measurements and dimensions of the aorta, constitute the output of the method of the present invention. In other words, the output of said method is quantitative subject-specific anatomy information.

Preferably, in certain embodiments of the present invention, segmentation of full heart or a fragment thereof, which may include aorta including aortic root, ascending aorta, aortic arch and descending aorta, left ventricle, right ventricle, left atrium, myocardium, pulmonary artery, and/or coronary arteries, is performed.

Valve calcification is a medical condition that occurs on one or more of the heart valves, wherein calcium deposits form and build up on the valve in the heart depending on the conditions of the patient. Calcification can be deposited along different parts of the heart but they are most typically found on the aortic valve in the heart which is called as aortic valve calcification. A region of the valve, preferably of the aortic valve, wherein calcium deposits are formed, is referred to as the calcified region.

Aortic stenosis is a disease that occurs as a result of valve calcification, which leads to the narrowing of the aortic root region, proximal to the exit of the left ventricle of the heart (i.e. the beginning of the aorta). A severe stenosis can result in death or stroke if not treated early. Due to narrowing, the blood flow in the narrowed region disturbs significantly, particularly in the aortic root region that is comprised of leaflets and the small portion of the coronary arteries. Due to calcification, the regions of the valve leaflets that are affected by the aortic stenosis are narrowed and lose their functioning, which is also referred to as the stenotic regions.

Computer vision is concerned with the automatic extraction, analysis and understanding of useful information from a single image or a sequence of images. In certain embodiments of the present invention, the computer vision method of the present invention comprises two steps: pre-processing step and segmentation step. In the pre-processing step, the images are first normalized by converting their intensity range (i.e., the range the $I(i,j)$ or $I(i, j, k)$ of the image may take) to a desired range of $[0, 1]$, and then thresholded to remove any background noise (i.e. all the $I(i,j)$ or $I(i, j, k)$ values below certain threshold are set to zero). In the segmentation step, the user clicks inside the aorta to create the initial seed points for a region growing algorithm. The region growing algorithm outputs the reconstructed aorta, which is then used as a mask to identify calcification regions. After processing, the user can visualize the reconstructions. In certain embodiments of the present invention, the images are CT images, for example 12 bit CT images, stored and processed as DICOM files, as known to the skilled person.

In certain embodiments of the present invention, The computer vision method comprises the steps of 1) reading of the grey value values of the image; 2) selection of the reference points 3) defining a global threshold (to this end, in the computer interface the user clicks on the aorta and defines the threshold value for the next processes; preferably, the value corresponding to that of the clicked region of aorta is taken); 4) growing a region from the reference points; and 5) binarization of the grown contours (wherein continuous grayscale value is converted to a boolean variable, i.e. 0 or 1). Figure 1 (lower panel, left) represents the segmentation system for computer vision calculations of the present invention as applied to the task of providing the three-dimensional representation of the geometry of the subject's heart and/or aorta. In certain embodiments, the medical imaging device measurements, for example MRI and/or CT images, are used as an input. Optionally in the methods of the present invention, initial user input may be needed, to indicate the aorta in the images. In certain embodiments of the present invention, this can be performed by using a user interface and, for example, performed by a double click on the aorta. In certain embodiments of the present invention, the input is sent to the processing unit. In certain embodiments of the present invention, processing unit uses computer vision algorithms to perform the segmentation. In certain embodiments, the results of the said segmentation are stored in a database and/or are provided to the user through a user interface.

Machine learning algorithms build a mathematical model based on sample data, known as "training data", in order to make predictions or decisions without being explicitly programmed to do so. In certain embodiments of the present invention, the machine learning method uses the images as well as output of the computer vision method, preferably with manual annotation of the heart components, as defined herein, as an input. As an output, a 2D mask that includes three labels: 0, 1, and for the background, aorta and calcification, respectively, is generated in the machine learning method of the present invention. Preferably, as an output, a 2D mask that includes eight labels: 1, 2, ..., 8 for the background, aorta including coronary arteries, left ventricle, myocardium, right ventricle, left atrium, pulmonary artery and calcification, respectively, is generated in the machine learning method of the present invention. In certain preferred embodiments, three, four six or ten labels can be used. The number of labels in certain preferred embodiments is not meant to be limiting, it is to be understood that the 2D mask may include also more than eight labels. In particular, larger number of labels would be required to describe components of the heart beyond aorta. In certain embodiments of the present invention, the machine learning method uses the images as well as output of computer vision method, as defined herein, as an input.

In certain embodiments of the present invention, the machine learning method is based on the U-Net model (Ronneberger, Olaf; Fischer, Philipp; Brox, Thomas (2015). "U-Net: Convolutional Networks for Biomedical Image Segmentation". arXiv:1505.04597) and comprises blocks of 2D convolutional layers. The said blocks are arranged in an encoder-decoder setting. Skip connections are utilized between each block. The U-Net architecture is a fully-connected 2D convolutional neural network (CNN) constructed for creating segmentation maps of input images. It is composed of a contracting encoder path and an expanding decoder path. The network has a varying depth depending on the application and each block of layers in the encoder path is connected to the corresponding block in the decoder path via skip connections. In certain embodiments the network has a depth of 5 and each block of layers in the encoder path is connected to the corresponding block in the decoder path via skip connections. In certain embodiments, the network has a depth of 4. A block of 3x3 convolutional layers followed by a rectified linear unit (ReLU) activation function and a 2x2 max-pooling layer is repeated in the contracting path. In the expanding path, the same block is repeated, but the max-pooling layer is removed and a 2x2 upsampling later is placed at the start of the block. In the method of the present invention, the input data is split into training, validating and testing sets. First, the training data is processed using data augmentation techniques, including random horizontal flips, random rotations, random scaling and random intensity shifts. These techniques are known to the skilled person. In certain embodiments, a dropout layer is included in the machine learning method of the present invention and early stopping is used to reduce the likelihood of overfitting.

Figure 1 (lower panel, right) represents segmentation system for artificial intelligence calculations of the present invention as applied to the task of providing the three-dimensional representation of the geometry of the subject's heart and/or aorta. In certain embodiments of the present invention, the medical imaging device measurements, for example MRI and/or CT images, are used as an input. In certain embodiments of the present invention, the method of the present invention relies on the user input. The user input is utilized in certain embodiments by the computer vision method. Thus, in certain embodiment the user input, which for example may be given through user interface, together with the input from the medical imaging device measurement, are sent to the processing unit. In certain embodiments of the present invention, the trained mathematical model as understood herein, is trained by the processing unit using the data stored in the database I. In certain embodiments, processing unit uses the trained mathematical model to define the requirements of the appropriate 3D geometry and, by using the data comprised in the database I, provide the three-dimensional representation of the geometry of the subject's heart and/or aorta to the processing unit, which finalizes the segmentation. In certain embodiments, the results of the said last step, namely three-dimensional representation of the geometry of the subject's heart and/or aorta, are stored in the database, and/or sent to the user through a user interface. Preferably, the user interface can be a web-based platform or a standalone executable.

The database I, as defined above and referred to in Figure 1, relates to a data structure comprising all the metadata in a structured manner. In certain embodiments, the data types stored in the database I are DICOM files from CT and MRI scanners. The CT files typically are 12-bit grayscale images, and the MRI files typically are 16-bit grayscale images. In certain embodiments of the present invention, the database comprises anonymized CT and MR images, and their respective aorta including coronary arteries, left ventricle, myocardium, right ventricle, left atrium, pulmonary artery regions reconstructed according to the methods of the present invention. In certain embodiments of the present invention, the database comprises anonymized CT and MR images, and their respective aorta and calcification regions reconstructed according to the methods of the present invention. In certain embodiment of the present invention, CT images are stored in the database as 12 bit CT images, and/or as DICOM format files.

The workflow described herein may also be used as an independent workflow for providing a subject-specific three-dimensional representation of the geometry of at least a portion of the heart and/or aorta. It can be combined with any other method, wherein access to the subject-specific three-dimensional representation of the geometry of at least a portion of the heart and/or aorta is useful. For example, it can be incorporated in the other methods of the present invention.

In the method of the present invention, the subject-specific three-dimensional representation of blood flow in at least the portion of the heart and/or aorta is provided according to the measurements of the blood flow in a subject. Any suitable experimental and/or diagnostic method can be used to obtain the said three dimensional representation of blood flow in at least the portion of the heart and/or aorta. Preferably, the said information in the method of the present invention is obtained by the means of 4D MRI. However, this should not be construed as limiting and other suitable methods also fall within the scope of the present invention. In another embodiment, the three dimensional representation of blood flow in at least the portion of the heart and/or aorta can be obtained by using the ultrasound methods. Typically, in the method of the present invention for planning a heart valve implantation in a subject as described herein, the subject-specific three-dimensional representation of blood flow in at least the portion of the heart and/or aorta is determined in a subject whereby no implanted heart valve is present. However, in certain embodiments of the present invention, for example in the cases wherein the second valve implantation is required, the subject-specific three-dimensional representation of blood flow in at least the portion of the heart and/or aorta may be determined experimentally in the presence of an implanted heart valve. In such cases, post operation blood flow characteristics can be determined by using a 4D MRI blood flow scan. In certain embodiments of the present invention, the post operation blood flow characteristics can be determined by using in vitro measurements, for example in phantoms, as described herein.

The further step of the method of the present invention comprises determining blood flow characteristics using a previously trained mathematical model in the presence and absence of an implanted heart valve based on the three-dimensional representation as described above, and varied size, design, implantation depth, and/or orientation of the implanted heart valve. As preferably to be understood herein, the blood flow characteristics determined in the presence and absence of an implanted heart valve are to be understood as comprising the blood flow characteristics determined in the presence of an implanted heart valve and the blood flow characteristics determined in the absence of an implanted heart valve. Preferably, if not explicitly described otherwise, the blood flow characteristics determined in the presence or absence of an implanted heart valve are to be understood as comprising the blood flow characteristics determined in the presence of an implanted heart valve or the blood flow characteristics determined in the absence of an implanted heart valve. The blood flow characteristics that are determined in the method of the present invention comprise blood flow patterns, velocity, retrograde flow rate, kinetic energy, vorticity, helicity, turbulence intensity, energy loss and/or shear stress. **In** certain preferred embodiments of the present invention, the blood flow characteristics that are determined in the method of the present invention comprise blood flow patterns, velocity, acceleration, retrograde flow rate, mean kinetic energy, turbulent kinetic energy, vorticity, helicity, turbulence intensity, energy loss, stroke work and/or shear stress.

As referred to herein, "velocity", or the velocity vector is to be understood as the vector representing the phase-averaged speed of the blood particles.

$$u_i = U_i + u_i{}'$$

$$(1)$$

wherein $u_i$ is the instantaneous velocity of a fluid particle, $U_i$ is the phase averaged velocity of a fluid particle, and $u_i'$ is the fluctuating velocity of a fluid particle,

As referred to herein, "mean kinetic energy", also referred to as MKE, is a function of square of the velocity, according to the formula:

$$MKE = u_i{}^2$$

$$(2)$$

As referred to herein, "fluctuating velocity" is defined as the root mean square of the velocity, according to the formula:

$$u_i' = \sqrt{<u_i>^2}$$

$$(3)$$

As referred to herein, "turbulent kinetic energy", also referred to as TKE, is a function of square of the fluctuating velocity, according to the formula:

$$TKE = u_i'^2$$

$$(4)$$

As referred to herein, "turbulence intensity", also referred to as TI, is defined as the ratio of fluctuating velocity to phase averaged velocity, according to the formula:

$$TI = \frac{u_i'}{U_i}$$

$$(5)$$

As referred to herein, "total kinetic energy", also referred to as E, is defined as the sum of turbulent kinetic energy and mean kinetic energy.

$$E = MKE + TKE$$

$$(6)$$

As referred to herein, "volumetric flow rate", also referred to as Q, is defined as the volume of the fluid (herein preferably blood or liquid modelling the blood) which passes through a certain point (e.g. the valve) per a unit of time.

$$Q = U_i * A$$

$$(7)$$

Where A is the cross-sectional area of the artery.

As referred to herein, "retrograde flow rate", also referred to as $Q_{ret}$, is defined as the volumetric flow rate in the case of reversed flow, and is negative.

$$Qret = Q < 0$$

$$(8)$$

As referred to herein, "vorticity" is defined as the curl of velocity vector.

$$w = \nabla \times U_i$$

$$(9)$$

As referred to herein, "helicity" is defined herein as the cosine of the angle between the velocity and the vorticity:

$$H = \frac{U_i . w}{|U_i| . |w|}$$

$$(10)$$

As referred to herein, "mean energy loss" is defined as energy dissipated into heat due to mean shear:

$$ELm = 2\vartheta S_{ij}S_{ij}$$

$$(11)$$

wherein $S_{ij}$ is defined as

$$S_{ij}=0.5((\partial U_i)/(\partial x_j)+(\partial U_j)/(\partial x_i))$$

$$(12)$$

As referred to herein, "turbulent energy loss" is defined as energy dissipated into heat due to turbulence:

$$ELt = 2\vartheta S_{ij}'S_{ij}'$$

$$(13)$$

wherein $S_{ij}'$ is defined as:

$$S_{ij}'=0.5((\partial u_i')/(\partial x_j)+(\partial u_j')/(\partial x_i))$$

$$(14)$$

As referred to herein, "shear stress" is defined as the gradients of the velocity profile:

$$VSS = \mu\left(\frac{\partial U_i}{\partial x_j} + \frac{\partial U_j}{\partial x_i}\right)$$

$$(15)$$

As referred to herein, "stroke work" is defined as the work done by the ventricle to eject a volume of blood.

$$SW=SV*MAP$$

$$(16)$$

wherein SV is the stroke work and MAP is the mean arterial pressure

In certain embodiments of the present invention, the blood flow characteristics as defined herein are temporally and/or spatially resolved. In other words, the blood flow characteristics as defined herein may be different in different points of the portion of the heart and/or aorta, and may be time dependent. For example, the blood flow characteristics may be different at different time points corresponding to different phases of the cardiac cycle. Further in certain embodiments, the said blood flow characteristics are determined in systolic and/or diastolic phase. Preferably, the blood flow characteristics as defined herein may be time-averaged and/or volume-averaged. In particular, the blood flow characteristics as determined by the previously trained mathematical model may be time averaged and/or volume averaged.

In certain embodiments of the methods of the present invention, the previously trained mathematical model is a machine learning-based method. Preferably, the previously trained mathematical model is a deep learning network. Deep learning network, which preferably corresponds to a set of algorithms introduced by machine learning methods, is understood and practiced as a class or subset of machine learning algorithms that uses multiple layers, preferably in a hierarchical manner, wherein the system progressively extracts higher level features from the raw input, which preferably is a database, and preferably a set of learning parameters. In a typical embodiment, the deep learning network comprises multiple layers. A deep learning network is typically trained by using a training dataset.

In certain embodiments of the present invention, the previously trained mathematical model, for example a deep learning network, relies on a training dataset comprising experimental blood flow characteristics in phantoms of at least the portion of the heart and/or aorta. Preferably, the training dataset may further comprise information on categorized anatomies, risk zones and risk regions. Said phantom of at least the portion of the heart and/or aorta is defined herein as a three-dimensional model, preferably obtained using additive manufacturing techniques (also referred to as 3D printing) according to the geometry of the heart and/or aorta of a subject, preferably using elastic and/or transparent material, more preferably using anatomically accurate, elastic, compliant, and/or transparent material. A non-limiting example of a suitable material for manufacturing of the said phantom is silicone. Silicone is herein defined as a material comprising polysiloxane polymers, according to the formula:

$$-(O-SiR_2)_n- \qquad \text{Formula 1}$$

wherein preferably each R is independently an alkyl group, wherein certain R can optionally be replaced by a di-valent alkylene group acting as a crosslinker attached to Si atom in another polysiloxane chain according to formula 1, and wherein n is an integer. In certain embodiments, phantoms described herein are obtained by 3D printing, in a process guided by the geometry of the heart and/or aorta of a subject. In certain embodiments, the said geometry may be obtained from a specific subject by using the methods of the present invention.

In certain embodiments, the said phantoms are used in the experimental model measurements of the blood flow, for example using the methods of 2D/3D Particle Imaging and/or Particle Tracking Velocimetry. The methods of Particle Imaging Velocimetry and Particle Tracking Velocimetry differ from each other both in the empirical measurements in the field, and the treatment of particles in the computational calculations the follow the experimental measurements. Particle Imaging Velocimetry is an optical imaging technique wherein the velocity field (in other words, spatially and temporally resolved fluid flow velocity) of an entire region within the flow is measured simultaneously by tracking clouds of tracer particles (in other words, tracing the assemblies of multiple independent tracer particles in an Eulerian flow field). Particle Tracking Velocimetry method is an optical imaging technique where the individual tracer particles are tracked to assess velocity information which allows to extract Lagrangian flow field within the region of interest.

As defined herein, preferably 2D/3D Particle Imaging and/or Particle Tracking Velocimetry are performed so that they comprise the following steps: calibration of the camera, pre-processing including detection of tracer particles, and processing including tracking of the individual particles or particle clouds.

In certain embodiments, the training dataset comprises the blood flow characteristics obtained from one or more subject(s). Preferably, the training dataset comprises the blood flow characteristics from more than 10, or more than 50, or preferably more than 100 subjects so that diverse blood flow characteristics (preferably including description of risk regions) are covered in the said training dataset. Preferably, corresponding risk regions and risk zones are also covered in the said training dataset. Further preferably, the training dataset comprises the blood flow characteristics from more than 10, or more than 50, or preferably more than 100 subjects so that diverse blood flow characteristics and risk assessment for post-heart valve replacement operation blood flow characteristics are covered in the said training dataset. The blood flow characteristics may be obtained from a subject by the means of a 4D MRI blood flow scan. However, this is not meant to be limiting and any method suitable for obtaining blood flow characteristics from the subject is applicable in the present invention.

Further preferably within the scope of the present invention the experimentally obtained data on blood flow characteristics from different patients with different geometry of at least a portion of the heart and/or aorta and different valve implantation parameters is used to account for any effects of variations of the valve implantation parameters on blood flow characteristics, preferably wherein patients can be categorized into bins of similar geometry of at least a portion of the heart and/or aorta. In other words, preferably wherein patients can be clustered into groups of similar geometry of at least a portion of the heart and/or aorta. The present inventors have found that preferably, in order to achieve the accuracy of the previously trained mathematical model of more than 90%, at least 65 patients of similar heart geometry and/or similar disease, for example aortic valve disfunction.

Preferably within the scope of the present invention, different geometry of at least a portion of the heart and/or aorta as accounted in the training set are categorized into several bins. Preferably, when the previously trained mathematical model is faced with prediction based on subject specific geometry of at least a portion of the heart and/or aorta, the previously trained mathematical model finds a bin that is the closest to the said subject-specific geometry of at least a portion of the heart and/or aorta and performs the said predictions based on the geometry of at least a portion of the heart and/or aorta as in the selected bin. Preferably, geometries of at least a portion of the heart and/or aorta categorized into different bins may also be referred to as categorized anatomies.

In certain embodiments, the training dataset comprises the blood flow characteristics obtained through simulation(s) which comprises blood flow velocities, coordinates of the fluid particles in different phases of a heartbeat. Preferably however, within the scope of the present invention the blood flow characteristics are not obtained through the simulations.

In certain embodiments, the said training dataset comprises the experimental blood flow characteristics obtained in the phantoms of at least the portion of the heart and/or aorta, determined in the presence of pathological conditions and/or implanted medical devices. In certain embodiments, the said training dataset comprises preferably the experimental blood flow characteristics obtained in the phantoms of at least the portion of the heart and/or aorta, determined in the presence or absence of pathological conditions and/or implanted medical devices. In certain embodiments, the said training dataset comprises the experimental blood flow characteristics obtained in the phantoms of at least the portion of the heart and/or aorta, determined in the presence of pathological conditions and/or implanted medical devices. In the method of the present invention, the valve of the phantom may be replaced with a device that simulates a stenotic valve. In further certain embodiments, the valve may be placed inside the phantom, so that the valve's behaviour can be studied experimentally. As described herein, the preferred methods suitable for such study are 2D/3D Particle Imaging and/or Particle Tracking Velocimetry. Preferably, in certain embodiments, the said training dataset comprises the experimental blood flow characteristics obtained in the phantoms of at least the portion of the heart and/or aorta, determined in the absence of pathological conditions and/or implanted medical devices. Further preferably, in certain embodiments, the said training dataset comprises the experimental blood flow characteristics obtained in the phantoms of at least the portion of the heart and/or aorta, determined in the absence of pathological conditions and/or implanted medical devices.

Preferably, within the scope of the present invention different valves are implanted in the same phantom, or the same valve is differently implanted in the same phantom so that the blood flow characteristics can be experimentally determined and account for any effects of variations of the valve implantation parameters, which preferably include but may not be limited to size, design, implantation depth and/or orientation of the implanted heart valve. To account for differences in the geometry of at least a portion of the heart and/or aorta, phantoms with different geometry of at least a portion of the heart and/or aorta are preferably used within the scope of the present invention.

Further preferably, in order to mimic the patient conditions when working with phantoms, preferably the subject's heart rate is mimicked by using a pulse duplicator and a ventricle assist device. Therefore, the blood inflow can be controlled and configured to be comparable to that of the patient under physiological conditions.

The next step of the method comprises determining the size, design, implantation depth and orientation of the implanted heart valve based on the determined blood flow characteristics in the presence and absence of the implanted heart valve. Determining the size, design, implantation depth and orientation of the implanted heart valve is herein understood as determining the optimal size, design, implantation depth and orientation of the implanted heart valve. Preferably, determining the size, design, implantation depth and orientation of the implanted heart valve can herein be understood as predicting post-implantation blood flow risks, determining patient specific heart valve implantation parameters such as size, implantation landing zone, commissural alignment/misalignment status and device type.

The obtained as described herein subject-specific three-dimensional representation of the geometry of at least a portion of the heart and/or aorta is used in another set of deep learning algorithms that calculate the blood flow characteristics in different conditions. The blood flow characteristics are preferably calculated in the presence and in the absence of the implanted heart valve. Further, the blood flow characteristics are determined based on a subject specific three dimensional representation of the geometry of at least a portion of the heart and/or aorta using a previously trained mathematical model, as described herein. However, it is further to be understood that determined blood flow characteristics in the presence and absence of the implanted heart valve may also be determined based on an experimentally determined subject-specific three dimensional representation of blood flow in at least a portion of the heart and/or aorta, for example based on 4D MRI data or on 3D Particle Tracking Velocimetry, or may be determined based on a subject specific three dimensional representation of the geometry of at least a portion of the heart and/or aorta using a previously trained mathematical model, as described herein.

Preferably, in the methods of the present invention the implanted heart valve refers to an aortic valve, however the aspects described herein may be generalized to other valve types, including tricuspid valve, pulmonic valve, and mitral valve. In certain embodiments, the blood flow characteristics are calculated for different implanted heart valve types and/or different implanted heart valve sizes and/or different implanted heart valve orientations and/or different implanted heart valve implantation depths. The output is valve performance score based on the post-operational hemodynamics prediction.

An implanted heart valve as referred to herein is an implantable medical device that is implanted into the heart of a patient suffering from a valve disease to replace one of the dysfunctional native heart valve of the patient. The replacement of the artificial valve is applied either via open heart surgery or minimally invasive heart valve replacement operations in the human heart and applied to one of the four valves, namely: tricuspid valve, pulmonic valve, mitral valve and aortic valve. The valves open and close with each heartbeat, allowing for blood to flow in and out of the heart and in the circulatory system. The main purpose of the valves is to keep blood flowing in one direction in and out and through the heart in order to regulate the flow of oxygenated and deoxygenated blood and prohibit the backflow of the blood. They also keep one-way blood flow from the heart into the circulatory system from the heart into the major blood vessels connected to it (such as the pulmonary artery and the aorta) and prohibit the backflow of the blood. One of the four heart valves can lose functioning/malfunction for a variety of reasons, such as diseases like aortic stenosis resulting from calcification of the heart valves. The narrowing of the valve opening due to stenosis can impede the flow of blood through the valve, which is called stenosis and/or let blood flow backwards (backflow of blood or leakage) through the valve, which is called regurgitation. If not treated, the situation may become more severe over time which may lead to death or stroke of the patient in several years. Both processes can increase the workload of heart substantially, put strain on the heart and may lead to serious health problems in the patient, including heart failure which eventually can lead to death. While in some cases of heart valve diseases, several early treatment options can be offered to the patient, for example dysfunctional heart valves can be treated with drugs or if detected early, the diseased region can be surgically repaired, the most common contemporary standard of care in the clinics for these patients typically involves a heart valve replacement with an implanted artificial heart valve, for example minimally invasive replacement operations.

In today's practice, different heart valves, such as mechanical, biological and tissue-engineered artificial heart valves are being used in the clinics for the treatment of heart valve dysfunction. Mechanical valves have various designs from different manufacturers that offer different types of valves, namely caged-ball, tilting-disc, and bileaflet valves. From the types of mechanical valves, bileaflet valves are the most common type of mechanical valves implanted to the patients in the clinics. The second type of artificial heart valves, biological valves also have different sizes and designs, and are mainly produced using animal tissue, e.g. bovine and pig tissue. The two types of biological valves are the tissue valves and the most commonly used bioprosthetic valves. The third type of artificial heart valve is the tissue-engineered heart valves, produced based on the anatomy of the patient. In recent years, tissue-engineered heart valves have been studied which have a potential to have a longer life time compared similar to the native valves. TAVI valves are typically produced using tissue-engineered valves. The implantation parameters which include valve design, valve size, valve orientation, valve implantation depth, and/or valve deployment thickness are the core of the pre-operational planning. Further preferably the implantation parameters which include valve design, valve size, commissural alignment/misalignment status, implantation landing zone, and/or valve deployment thickness can be the core of the pre-operational planning.

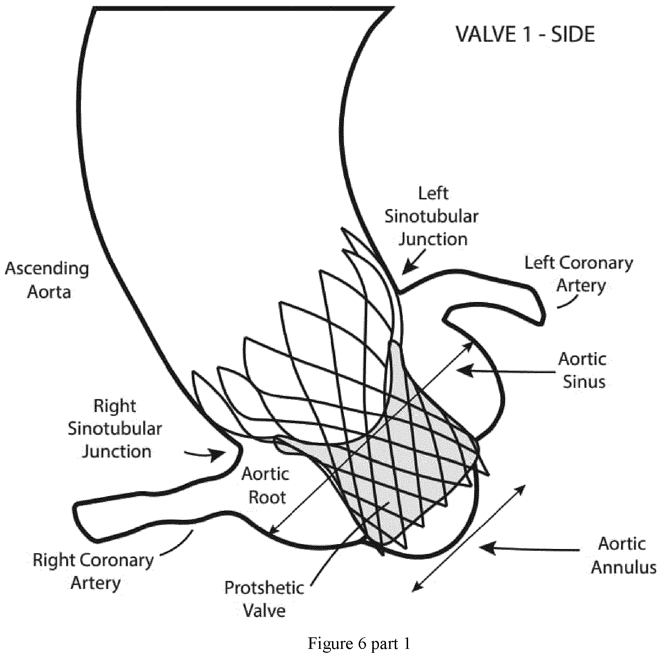

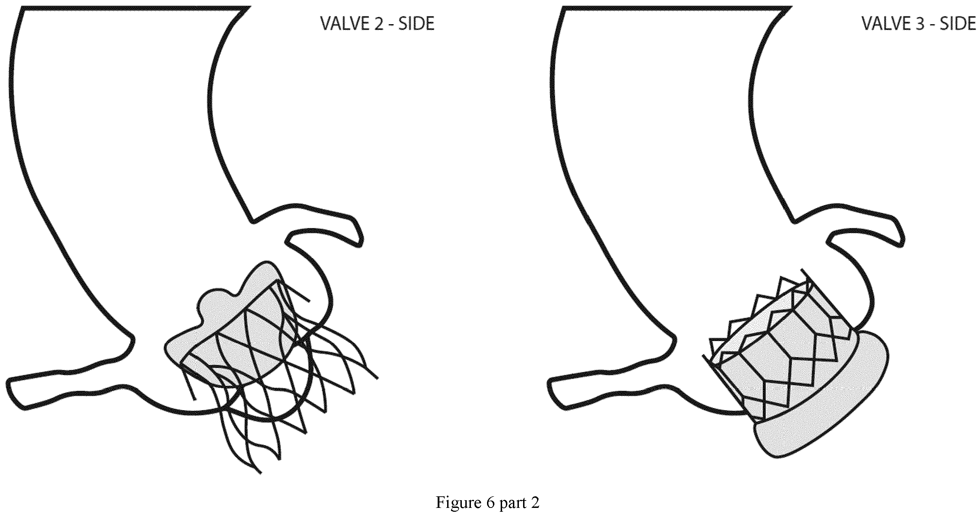

As defined herein, valve design relates to different possible shapes and structures of the valve, which are known to the skilled person in the art. Examples valve designs are depicted in Figure 6. As it is known to the skilled persons, central valve designs allow for varying valve sizes and valve deployment thicknesses.

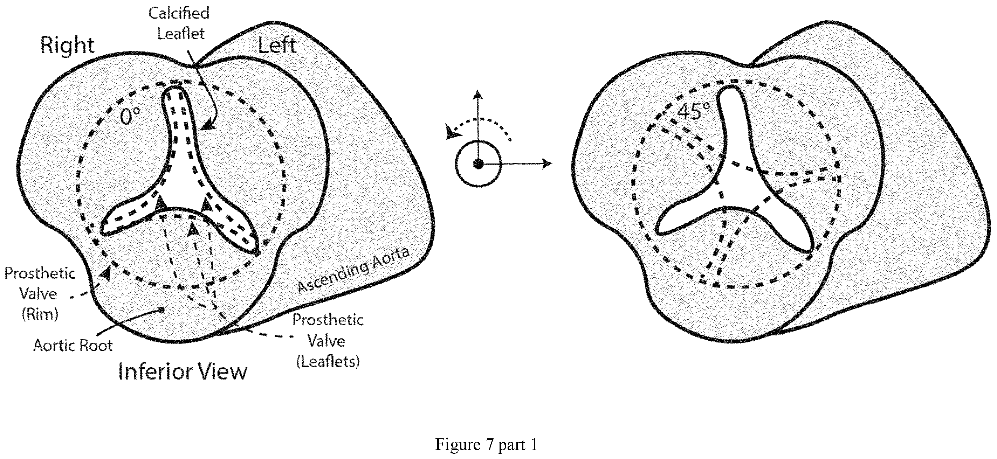

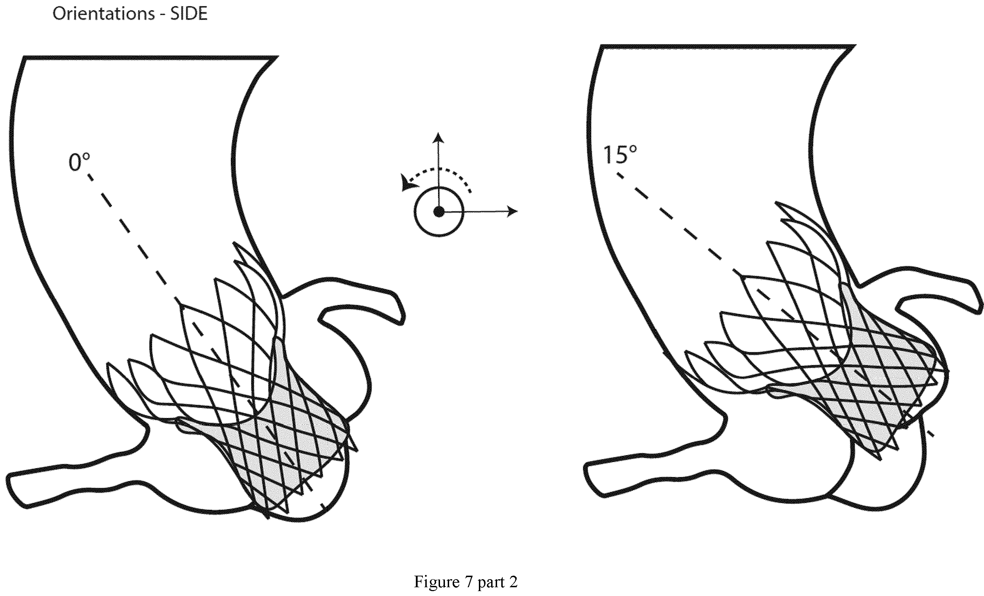

Valve orientation is herein defined as an angle with respect to the native valve orientation, as depicted in Figure 7.

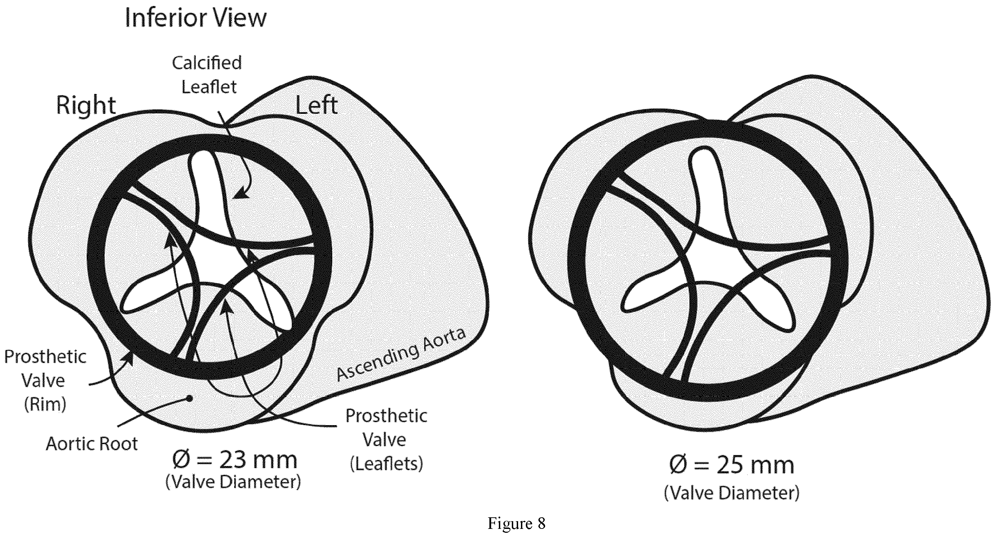

Valve size is defined herein as valve dimensions, in preferred embodiment valve diameter. In certain embodiments of the present invention, different valve designs may be preferably defined by different dimension parameter(s), referred to herein collectively as valve size. As it is known to the skilled person, certain valve designs allow for valve expansion. That means, valve size can be regulated in certain valve designs. Examples of different valve sizes are shown in Figure 8.

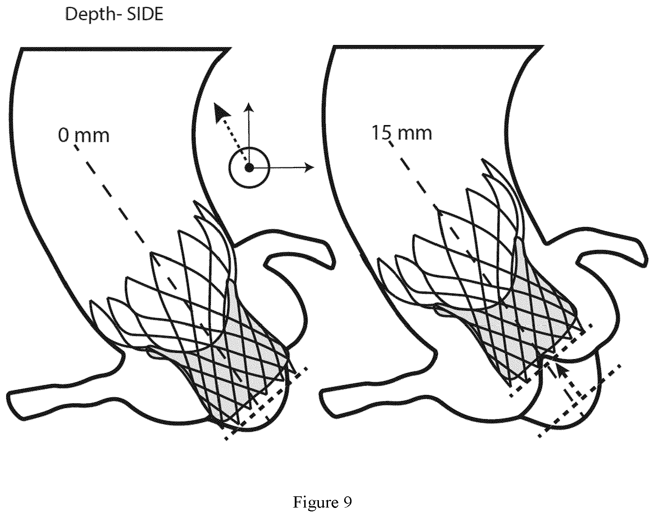

Valve implantation depth is defined herein as position of valve alongside the aorta with respect to the annulus, as depicted in Figure 9.

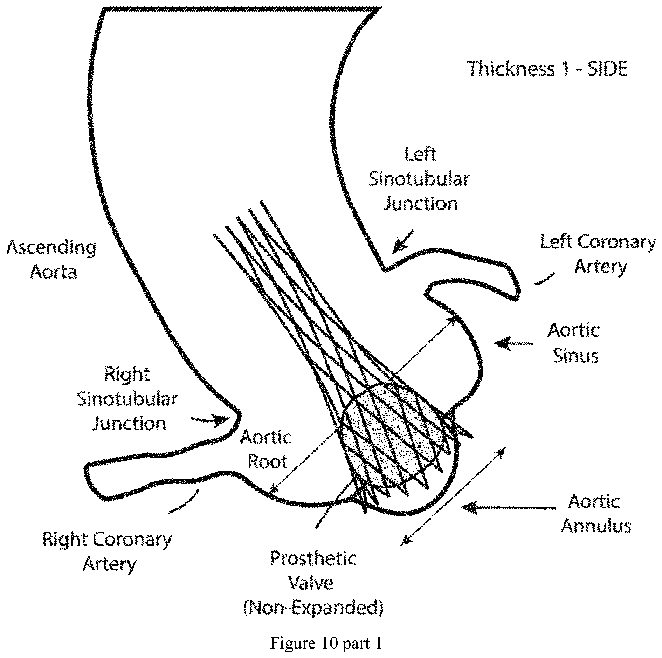

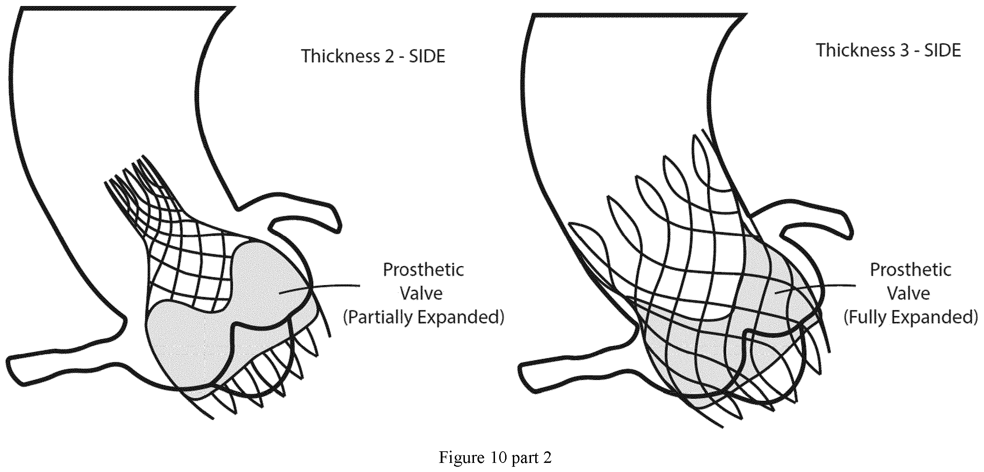

Valve deployment thickness is defined herein as the extent to which a valve of a design that allows regulation of valve size is expanded. Examples are depicted in Figure 10.

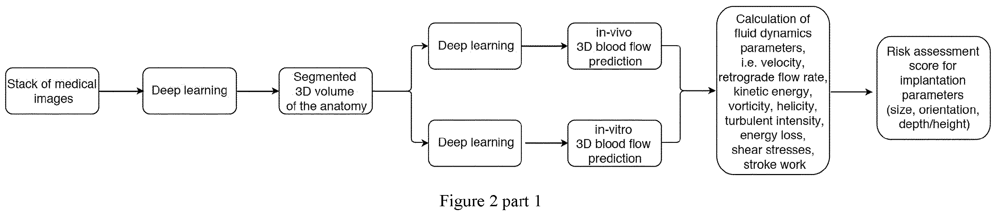

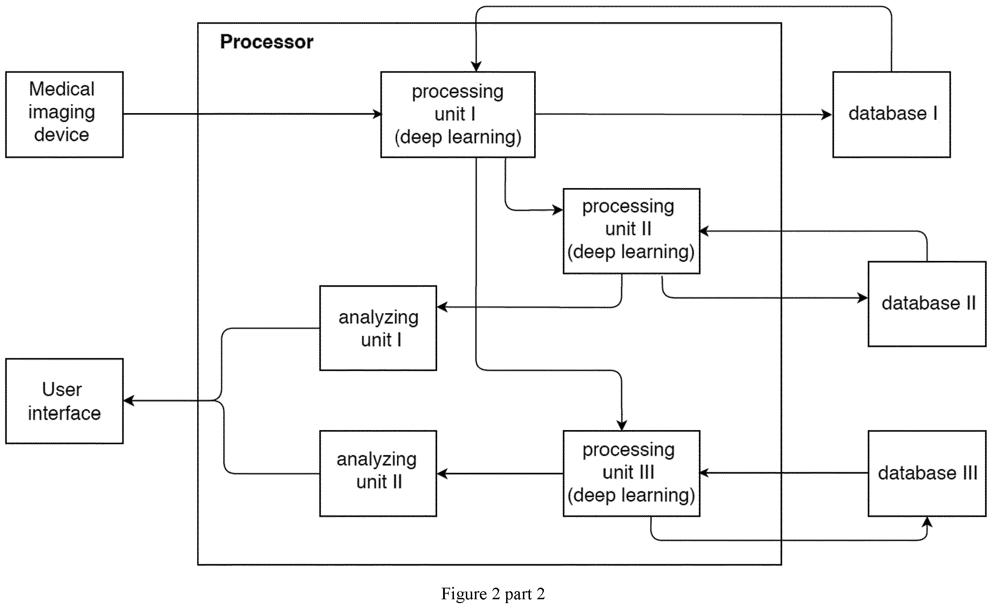

In certain embodiments, another set of deep learning algorithms that calculate the blood flow characteristics in different conditions comprises valve performance score system. Figure 2 (top panel) describes the workflow that allows for calculating the valve performance score, which is defined as the flow abnormalities due to high shear stress, turbulence intensity and energy loss. As preferably noted herein, the said flow abnormalities may also be due to different factors. In certain embodiments, the workflows as described in Figure 1 that output the subject-specific three-dimensional representation of the geometry of at least a portion of the heart and/or aorta may be also incorporated into the workflow for calculating the valve performance score. In certain embodiments, the processing unit I uses deep learning for generating the subject-specific three-dimensional representation of the geometry of at least a portion of the heart and/or aorta using the input from Medical imaging device, and sends it to the database I. In certain embodiments, the 4D MRI data and/or CT data are input from the Medical Imaging device, preferably either directly from the CT and MRI scanners or from the hospital data management PACS system . Database I stores the subject-specific three-dimensional representation of the geometry of at least a portion of the heart and/or aorta. Depending on the input, processing unit I may also retrieve the subject-specific three dimensional representation of the geometry of at least a portion of the heart and/or aorta from Database I by using the previously trained mathematical model. The processing unit II takes the subject-specific three-dimensional representation of the geometry of at least a portion of the heart and/or aorta from processing unit I as input and uses another set of deep learning algorithms that calculate the blood flow characteristics. In certain embodiments, calculated blood flow characteristics are selected from velocity, retrograde flow rate, kinetic energy, vorticity, helicity, and viscous shear stresses. The calculated blood flow characteristics are then sent to database II. Processing unit I provides processing unit III with the subject-specific three dimensional representation of the geometry of at least a portion of the heart and/or aorta. In certain embodiments of the present invention, the experimental blood flow characteristics measured in phantoms are provided to the processing unit III. Preferably, the experimental blood flow characteristics are measured by the means of 3D Particle Tracking Velocimetry and/or Particle Imaging Velocimetry. Processing unit III then calculates additional blood flow characteristics in the presence and in the absence of the implanted heart valve, e.g. turbulent intensity, energy loss, turbulent viscous dissipation and Reynolds shear stresses. In certain embodiments, analysing unit(s) calculate(s) valve performance score (also referred to as risk assessment score) based on the calculated 3D blood flow characteristics. In particular embodiments, analysing unit I relies on 3D blood flow characteristics obtained in vivo, while unit II relies on 3D blood flow characteristics obtained in vitro.

In certain embodiments of the present invention the size, design, implantation depth and/or orientation of the implanted heart valve are determined for a subject-specific three-dimensional representation of the geometry of at least a portion of the heart and/or aorta of a subject to minimize pressure drop, regurgitation volume, turbulence intensity, shear stress regions, stagnation regions and/or flow separation regions.

As referred to herein, "regurgitation volume" is defined as the negative fluid volume that has flown towards the left ventricle (see equation (8)).

As referred to herein, "(high) shear stress regions" are defined as regions wherein shear stress, as defined in equation (15) is high.

As referred to herein, "stagnation regions" are defined as a region wherein the local velocity of fluid is zero.

As referred to herein, "flow separation regions" are defined as the detachment of the boundary layer from a surface. A boundary is defined herein as the artery wall, in particular aorta wall.

In another aspect, the present invention relates to a method for determining performance of an implanted heart valve. In the first step of the said method, a subject-specific three-dimensional representation of the geometry of at least a portion of the heart and/or aorta of a subject, is provided as described herein.

In certain embodiments of the present invention, the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

In the next step of the method, a subject-specific phantom of at least the portion of the heart and/or aorta is obtained. In certain embodiments of the present invention, the subject specific phantom is obtained by using additive manufacturing techniques. In certain embodiments, preferably optically transparent elastic silicone phantoms are obtained. Silicone is defined herein as a material comprising a polymer according to Formula 1. In certain embodiments, the subject-specific phantom is manufactured by using additive manufacturing techniques according to the three-dimensional representation of the geometry of at least a portion of the heart and/or aorta of a subject, as obtained according to the methods of the present invention. Preferably, the three-dimensional representation of the geometry of at least a portion of the heart and/or aorta of a subject is provided in form of an .stl file format, also known as stereolitography file format.

Any type of silicone suitable for additive manufacturing techniques can be used in the present invention. Preferably, the silicone is transparent and its physical properties should be in the range corresponding to the properties of the heart tissue. In a non-limiting example embodiment, the silicone useful in the methods of the present invention has a hardness (Shore A) of 40, tensile strength of 971 psi, tensile strength of 6.7 MPa, elongation at break of 400 %, tear strength of 150 ppi, tear strength of 27 N/mm (according to ISO 34 cutter measurement), and Specific Gravity of 1.09. Herein, specific gravity of silicone is defined as the ratio of the weight of a given piece of silicone to the weight of an equal volume of water at a specified temperature.

Another step of the method of the present invention includes, in certain embodiments, using optical imaging techniques to obtain experimental blood flow characteristics in the subject-specific phantom of at least the portion of the heart and/or aorta in the presence and absence of the implanted heart valve. (Gülan, U. et al. Investigation of atrial vortices using a novel right heart model and possible implications for atrial thrombus formation. Scientifc Reports 7, 16772, https://doi.org/10.1038/s41598-017-17117-3 (2017); Gülan, U.; Saguner, A.M.; Akdis, D.; Gotschy, A.; Tanner, F.C.; Kozerke, S.; Manka, R.; Brunckhorst, C.; Holzner, M.; Duru, F. Hemodynamic Changes in the Right Ventricle Induced by Variations of Cardiac Output: A Possible Mechanism for Arrhythmia Occurrence in the Outflow Tract. Sci. Rep. 2019, 9, 100). Preferably, the optical imaging techniques comprise 2D/3D Particle Imaging and/or Particle Tracking Velocimetry. (Gülan, U. et al. Experimental study of aortic flow in the ascending aorta via particle tracking velocimetry. Experiments in Fluids 53, 1469-1485 (2012))

The optical imaging technique comprises components of high-speed camera, an image splitter, a light source (laser), and a working fluid is a mixture of glycerol, water and NaCl with a kinematic viscosity matching the refractive index of the silicone phantom. Both Particle Imaging Velocimetry (PIV) and Particle Tracking Velocimetry (PTV) are utilized as optical imaging techniques. The fluid dynamics analysis comprises Eulerian and Lagrangian tracking of particles in the region of interest to assess velocity, acceleration, vorticity, helicity, mean kinetic energy, turbulent kinetic energy, laminar viscous dissipation, turbulent viscous dissipation, irreversible energy loss, viscous shear stress, Reynolds shear stress and wall shear stress. In certain embodiments, the output shows the impact of the individual valve design on the blood flow in the left ventricle and aorta, i.e. aortic root, ascending aorta, aortic arch, and descending aorta of many different anatomies. In certain embodiments, the fluid dynamics parameters are shown in qualitative and quantitative tables and plots. In the present invention, different valve prototypes can be examined so that the subject-specific valve design can be improved.

As referred to herein, "laminar viscous dissipation" is defined as mean energy loss, according to equation (11).

As referred to herein, "turbulent viscous dissipation" is defined as turbulent energy loss, according to equation (13).

As referred to herein, "irreversible energy loss" is defined as the sum of mean energy loss and turbulent energy loss.

As referred to herein, "viscous shear stress" is shear stress defined according to equation (15).

As referred to herein, "Reynolds shear stress" is defined as

$$RSS = \rho u_{i'}u_{j'}$$

$$(17)$$

As referred to herein, "wall shear stress" is defined as

$$WSS = \mu(\partial U i \partial x j)$$

$$(18)$$

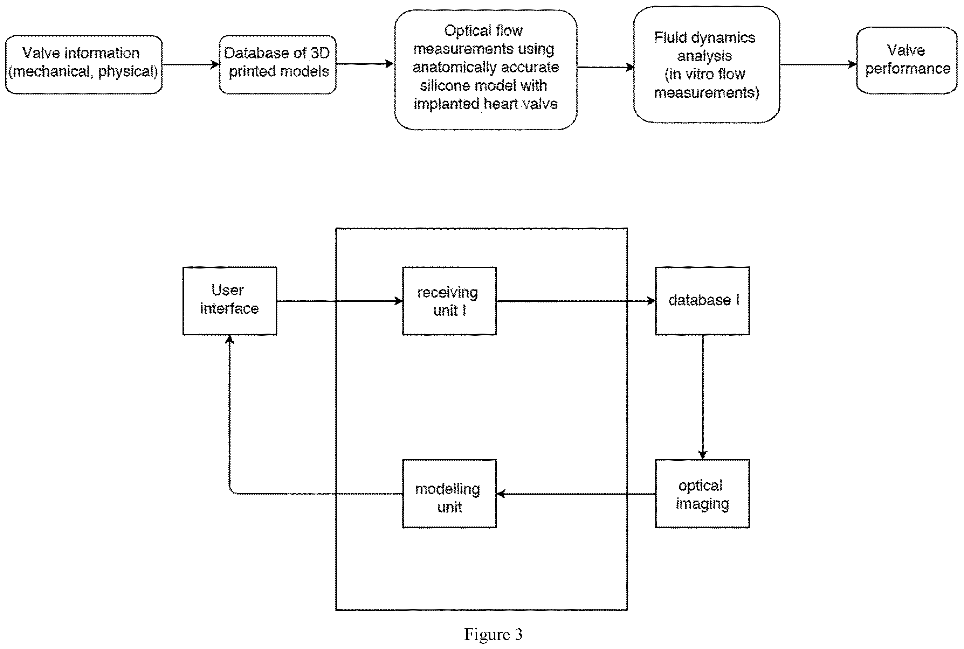

Figure 3 shows a workflow implementing the method for determining performance of an implanted heart valve of the present invention. In certain embodiments of the present invention, the user selects the valve properties, including the size, design, implantation depth and/or orientation of the implanted heart valve. In certain embodiments of the invention, receiving unit I receives the subject specific information from the user interface, and an algorithm retrieves appropriate subject-specific three-dimensional representation of the geometry of at least a portion of the heart and/or aorta from the Database I. Any algorithm capable of performing such a selection can be used here. In a preferred embodiment, computer vision and/or deep learning methods, as described herein, are used. Next, in certain embodiments subject-specific phantoms are obtained according to the approaches described herein, and optical imaging measurements are done as described herein, relying on the input from receiving unit I. It is to be understood that, according to the methods of the present invention, the size, design, implantation depth, deployment thickness and/or orientation of the implanted heart valve are to be varied in the course of the measurements. Based on the collected data, the Modelling Unit calculates the fluid dynamics parameters of velocity, retrograde flow rate, kinetic energy, vorticity, helicity, turbulent intensity, energy loss and shear stresses. Preferably, the Modelling Unit calculates the fluid dynamics parameters of velocity, retrograde flow rate, kinetic energy, vorticity, helicity, turbulent intensity, energy loss, shear stresses, risk regions and/or risk zones. The output in the form of the valve performance for different conditions is provided to the user interface.

Herein, determined valve performance of the implanted heart valve comprises pressure drop, regurgitation volume, turbulence intensity, high shear stress regions, stagnation regions and/or flow separation regions. Preferably, determined valve performance of the implanted heart valve comprises further risk regions and/or risk zones.

The blood flow characteristics that are determined experimentally in the method of the present invention comprise blood flow patterns, velocity, retrograde flow rate, kinetic energy, vorticity, helicity, turbulence intensity, energy loss and/or shear stress. In certain embodiments of the present invention, the experimental blood flow characteristics are temporally and/or spatially resolved. In further embodiments, the experimental blood flow characteristics are determined in systolic and/or diastolic phase. In certain embodiments of the present invention, velocity and regurgitation volume can be measured by using 4D MRI flow scans.

In another aspect, the present invention relates to a method for determining subject-specific blood flow characteristics for at least a portion of the heart and/or aorta.

In the first step of the said method a subject-specific three-dimensional representation of the geometry of at least the portion of the heart and/or aorta and of blood flow therein is provided.

The subject-specific three-dimensional representation of the geometry of at least the portion of the heart and/or aorta is provided according to the methods described herein. Preferably, the three-dimensional representation of the geometry of the subject's heart and/or aorta is obtained from MRI and/or CT imaging as described herein, preferably wherein the MRI and/or CT images are automatically quantified and segmented by using a machine learning-based method as described herein, preferably wherein the machine learning-based method comprises a computer vision method, as described herein.

In the method of the present invention, the subject-specific three-dimensional representation of blood flow in at least the portion of the heart and/or aorta is provided according to the measurements of the blood flow in a subject. Any suitable experimental and/or diagnostic method can be used to obtain the said three-dimensional representation of blood flow in at least the portion of the heart and/or aorta. Preferably, the said information in the method of the present invention is obtained by the means of 4D MRI. However, this should not be construed as limiting and other suitable methods also fall within the scope of the present invention. In another embodiment, the three-dimensional representation of blood flow in at least the portion of the heart and/or aorta can be obtained by using the ultrasound methods.

In certain embodiments of the present invention, Database 1 comprises anonymized CT and MR images, and their respective reconstructed aorta and calcification regions. Preferably, in certain embodiments the said anonymized CT and MR images are the images of the whole heart, including right/left ventricles, atriums, coronary arteries, pulmonary artery, myocardium. In certain embodiments of the present invention Database 2 contains anonymized 4D MRI flow data and its parameters.

The further step of the method of the present invention involves determining the blood flow characteristics based on the three-dimensional representation of the geometry of at least the portion of the heart and/or aorta and of blood flow therein obtained as described above using a previously trained mathematical model. The obtained as described herein subject-specific three-dimensional representation of the geometry of at least a portion of the heart and/or aorta is used in another set of deep learning algorithms that calculate the blood flow characteristics. However, in certain embodiments of the present invention the blood flow characteristics may be determined further using an experimentally determined subject-specific three dimensional representation of blood flow in at least a portion of the heart and/or aorta, for example based on 4D MRI data, as an additional input for the previously trained mathematical model.

In certain embodiments of the methods of the present invention, the previously trained mathematical model is a machine learning-based method. Preferably, the previously trained mathematical model is a deep learning network. Deep learning network, which preferably corresponds to set of algorithms introduced by machine learning methods, is understood and practiced as a class or subset of machine learning algorithms that uses multiple layers, preferably in a hierarchical manner, wherein the system progressively extracts higher level features from the raw input, which is preferably a database, and preferably a set of deep learning parameters. In a typical embodiment, the deep learning network comprises multiple layers. A deep learning network is typically trained by using a training dataset.

In the method of the present invention, the training dataset used to train a previously trained mathematical model is assembled as described above. In certain embodiments, the previously trained mathematical model relies on a training dataset comprising experimental blood flow characteristics in phantoms of at least the portion of the heart and/or aorta, as disclosed herein. The said phantoms of at least the portion of the heart and/or aorta in certain embodiments are three-dimensional models, preferably obtained using additive manufacturing techniques according to the geometry of the heart and/or aorta of a subject, preferably using elastic and/or transparent material, more preferably using elastic, compliant, and/or transparent material, as disclosed herein. In certain embodiments of the present invention the experimental blood flow characteristics in the said phantoms are obtained by using optical imaging techniques, preferably 2D/3D Particle Imaging and/or Particle Tracking Velocimetry. In certain embodiments of the invention, the training dataset comprises the experimental blood flow characteristics in the phantoms of at least the portion of the heart and/or aorta, determined in the presence or absence of pathological conditions and/or implanted medical devices, preferably determined in the presence of pathological conditions and/or implanted medical devices, as disclosed herein. In certain further embodiments, the training dataset comprises blood flow characteristics obtained from one or more subject(s) and/or through simulation(s), as disclosed herein.

As defined herein, the blood flow characteristics comprise three-dimensional blood flow patterns, velocity, retrograde flow rate, kinetic energy, vorticity, helicity, turbulence intensity, stroke work, energy loss and/or shear stress. In certain embodiments of the present invention, the blood flow characteristics are temporally and/or spatially resolved. In further certain embodiments of the present invention the blood flow characteristics are determined in systolic and/or diastolic phase.

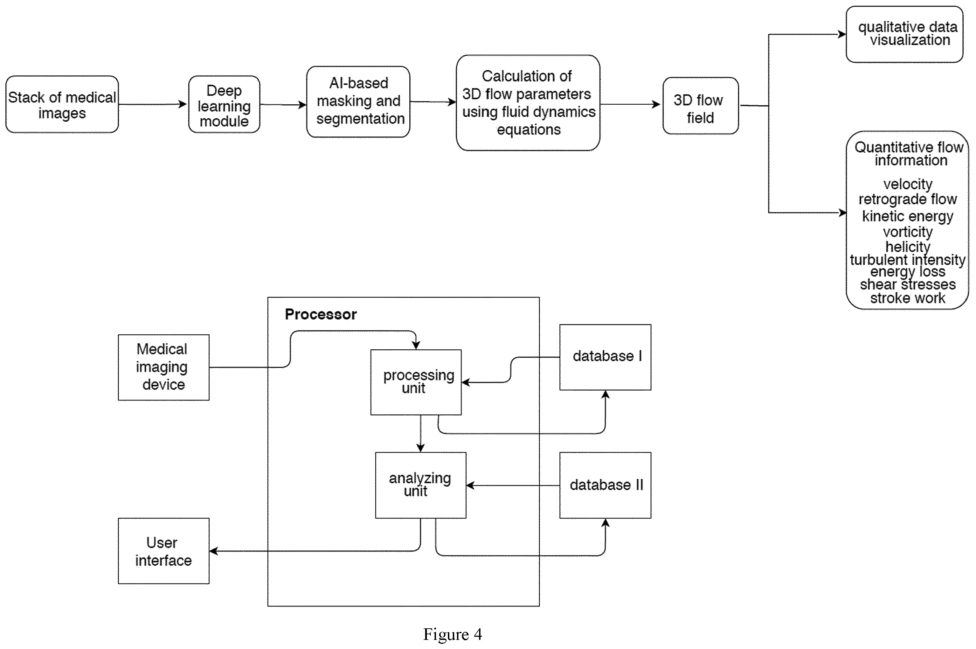

Figure 4 represents an implementation of the workflow of the method for determining subject - specific blood flow characteristics of the method of the present invention. Herein, input is a stack of medical images from MRI, CT, ultrasound or 4D MRI provided from a medical imaging device. Different types of data can be provided and/or consolidated in the method of the present invention. In certain embodiments, the processing unit receives provided medical images. In certain embodiments, three dimensional representation of the geometry of at least a fragment of the heart and/or aorta is provided as described above to the processing unit. Depending on the input, processing unit may in certain embodiments of the invention also retrieve the subject-specific three dimensional representation of the geometry of at least a portion of the heart and/or aorta from Database I by using the previously trained mathematical model. Said three dimensional representation of the geometry of at least a fragment of the heart and/or aorta is in certain embodiments then used to process the experimental blood flow characteristics by masking the respective experimental or analytical data (preferably experimental data), for example 4D MRI data or ultrasound data, to extract a three-dimensional representation of the blood flow in at least a fragment of the heart and/or aorta. In certain embodiments of the present invention, the said three-dimensional representation of the blood flow in at least a fragment of the heart and/or aorta is then used by the processing unit to determine the blood flow characteristics, represented as a 4D velocity vector field is created in the physical coordinate system using a previously trained mathematical model, trained based on the data in the database I. In certain embodiments, calculated blood flow characteristics are selected from velocity, retrograde flow rate, kinetic energy, vorticity, helicity, and viscous shear stresses. In certain embodiments of the present invention, this task is performed by analysing unit. The calculated blood flow characteristics are then sent to database II. In certain embodiments of the present invention, the so-obtained results are sent to the user through an interface. As understood herein, the interface can be a web-based interface, or a standalone executable software. In certain embodiments, calculated blood flow characteristics are selected from velocity, retrograde flow rate, kinetic energy, vorticity, helicity, and viscous shear stresses.

The workflow described herein may also be used as an independent workflow for providing preferably subject-specific blood flow characteristics based on three-dimensional representation of the geometry of at least a portion of the heart and/or aorta, and optionally on three-dimensional representation of the blood flow in at least a portion of the heart and/or aorta. It can be combined with any other method, wherein access to the said blood flow characteristics preferably in at least a portion of the heart and/or aorta is useful. For example, it can be incorporated in the other methods of the present invention.

In another aspect, the present invention relates to a system for determining subject-specific blood flow characteristics. In certain embodiments, the system of the present invention comprises at least one computer.

The said computer is configured to receive a subject-specific three-dimensional representation of the geometry of at least a portion of the heart and/or aorta. The subject-specific three-dimensional representation of the geometry of at least a portion of the heart and/or aorta may be provided from a database. In another embodiment, the subject-specific three-dimensional representation of the geometry of at least a portion of the heart and/or aorta is provided from a medical imaging device, optionally upon initial processing of the data as described herein.

In certain embodiments of the present invention, the three-dimensional representation of the geometry of the subject's heart and/or aorta is obtained from MRI, ECHO, and/or CT imaging, preferably from MRI and/or CT imaging, preferably wherein the MRI and/or CT images are automatically quantified and segmented by using a machine learning-based method, preferably wherein the machine learning-based method comprises a computer vision method.

Optionally, in certain embodiments the system of the present invention is configured to receive information about size, design, implantation depth and/or orientation of an implanted heart valve. The meaning of these parameters is as disclosed herein.

In certain embodiments of the present invention, the system of the present invention is configured to determine the blood flow characteristics based on the three-dimensional representation of the geometry of at least a portion of the heart and/or aorta and optionally on size, design, implantation depth, deployment thickness, and/or orientation of the implanted heart valve by using a previously trained mathematical model. In certain preferred embodiments of the present invention, the system of the present invention is configured to determine the blood flow characteristics based on the three-dimensional representation of the geometry of at least a portion of the heart and/or aorta and optionally on size, design, implantation depth, deployment thickness, orientation of the implanted heart valve, risk regions and/or risk zones by using a previously trained mathematical model.

In certain embodiments of the system of the present invention, the previously trained mathematical model is a machine learning-based method. Preferably, the previously trained mathematical model is a deep learning network. Deep learning network preferably corresponds to set of algorithms introduced by machine learning methods, and is understood and practiced as a class or subset of machine learning algorithms that uses multiple layers preferably in a hierarchical manner, wherein the system progressively extracts higher level features from the raw input, which preferably is a database, and preferably a set of learning parameters. In a typical embodiment, the deep learning network comprises multiple layers. A deep learning network is typically trained by using a training dataset. In certain embodiments of the system of the present invention, the previously trained mathematical model, preferably the deep learning network, is implemented using GPU-based cloud computing.

In the system of the present invention, the training dataset used to train a previously trained mathematical model is assembled as described above. In certain embodiments, the previously trained mathematical model relies on a training dataset comprising experimental blood flow characteristics in phantoms of at least the portion of the heart and/or aorta, as disclosed herein. The said phantoms of at least the portion of the heart and/or aorta in certain embodiments are three-dimensional models, preferably obtained using additive manufacturing techniques according to the geometry of the heart and/or aorta of a subject, preferably using elastic and/or transparent material, more preferably using elastic, compliant, and/or transparent material, as disclosed herein. In certain embodiments of the present invention the experimental blood flow characteristics in the said phantoms are obtained by using optical imaging techniques, preferably 2D/3D Particle Imaging and/or Particle Tracking Velocimetry. In certain embodiments of the invention, the training dataset comprises the experimental blood flow characteristics in the phantoms of at least the portion of the heart and/or aorta, determined in the presence or absence of pathological conditions and/or implanted medical devices, as disclosed herein. In certain embodiments of the invention, the training dataset comprises the experimental blood flow characteristics in the phantoms of at least the portion of the heart and/or aorta, determined in the presence of pathological conditions and/or implanted medical devices, as disclosed herein. In certain further embodiments, the training dataset comprises blood flow characteristics obtained from one or more subject(s) and/or through simulation(s), as disclosed herein.

As defined herein, the blood flow characteristics comprise three-dimensional blood flow patterns, velocity, retrograde flow rate, kinetic energy, vorticity, helicity, turbulence intensity, stroke work, energy loss and/or shear stress. In certain embodiments of the present invention, the blood flow characteristics are temporally and/or spatially resolved. In further certain embodiments of the present invention the blood flow characteristics are determined in systolic and/or diastolic phase.

**Brief description of Figures**

Figure 1 represents the workflow and its implementation used for providing a subject-specific three-dimensional representation of the geometry of at least the part of the heart and/or aorta of the present invention.

Figure 2 represents the workflow and its implementation used for planning a heart valve implantation in a subject according to the present invention.

Figure 3 represents the workflow and its implementation used for determining performance of an implanted heart valve according to the present invention.

Figure 4 represents the workflow and its implementation used for determining subject-specific blood flow characteristics for at least a portion of the heart and/or aorta according to the present invention.

Figure 5 depicts an example of an inserted prosthetic valve in the aorta. The prosthetic valve replaces the native valve of the patient.

Figure 6 shows different valve designs in the same patient specific anatomy. Left: implanted prosthetic valve design #1, Middle: implanted prosthetic valve design #2, Right: implanted prosthetic valve design #3.

Figure 7 depicts the orientation of the implanted heart valve in the same patient-specific anatomy. Top panel shows the orientation in the inferior view. The prosthetic valve may be placed between 0-120 degrees compared to the native valve. Top left, 0 degrees; top right, 45 degrees of implantation. Bottom panel shows the orientation on the side view. The prosthetic valve can be placed within a few 10s of degrees in order to optimize the blood flow along the ascending aorta. Bottom left, 0 degrees; bottom right, 15 degrees.

Figure 8 illustrates the implantation of different valve sizes for the same valve design in the same patient-specific anatomy. Left, 23 mm diameter valve; Right, 25 mm diameter valve.

Figure 9 shows the valve implantation depths for the same patient-specific anatomy. Left, 0 mm; right, 15 mm depth of implantation with respect to the annulus.

Figure 10 shows the valve deployment thicknesses in the same patient-specific anatomy, Left, non-expanded; Middle, partially expanded; Right, fully expanded.

Figure 11 depicts the premilinary results of the correlation between Mean Kinetic Energy (MKE) obtained via in vivo MRI measurement and the one obtained via invitro prediction for both before the actual TAVI implantation and after the TAVI operation

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

**Examples**

Example 1

Figure 1 represents an example of the image segmentation process in a patient-specific anatomy. The input is a stack of medical images from an MRI or CT scanner, which are converted into a pre-processed volume and are anonymized in the software. The 2D segmentation is performed based on machine learning and computer vision. The user is not permitted to continue the process without the anonymization step. The volume is segmented first using either of the two approaches: Computer vision or machine learning. While the machine learning method is much quicker, it requires powerful computers therefore the selection is left to the user. Depending on the user preferences, either cloud supported machine learning tool or offline computer vision tool provides the segmented anatomies.

The computer vision tool takes the anonymized pre-processed volumes. In this tool, initial user input is needed. The user scrolls the medical image to locate the aorta and make at least two clicks on the aorta. The processing unit takes the inputs and uses computer vision algorithms to do segmentation of full aorta (root, ascending, arch, descending), calcified regions, and valve leaflets including the stenotic region. The raw image and segmented results are stored in a database. The output (3D reconstructed aorta, calcification and stenosis measurements, and size of the aorta) are sent to the user through an interface. The user can scroll through different 2D slices and analyze the dimensions of the aorta, as well as its geometry, and calcified regions throughout the 3D volume. Additionally, an option is added for the user to prepare the geometry for 3D printing purposes.

The database from Computer Vision tool and/or manually segmented medical images is used to feed the machine learning module. This module is fully automated and does not require any user input. The user uploads the stack of medical images gathered from the medical imaging device measurement. The processing unit uses the trained model to find the anatomy needs and sends it to the database. Database sends the required anatomy as a result. The output (3D reconstructed aorta, calcification and stenosis measurements, and size of the aorta as well as other components of the heart) is stored in the database and also are sent to the user through an interface. Similar to the Computer Vision tool, the user can scroll through different 2D slices and analyze the dimensions of the aorta, as well as its geometry, and calcified regions throughout the 3D volume. Additionally, an option is added for the user to prepare the geometry for 3D printing purposes.

Example 2

Figure 2 shows an example of the pre and post operational flow. The input is a stack of medical images from medical devices, such as CT and MRI. The images are anonymized before moving to the next steps. Deep learning algorithms segment the image, as in Figure 1. The segmented algorithm is processed in two ways: 1) using a deep learning model created with the in-vivo 4D-MRI database and 2) using a deep learning model created using the in-vitro optical imaging database. Results of the both models calculate the fluid dynamics parameters such as velocity, retrograde flow rate, kinetic energy, vorticity, helicity, turbulence intensity, energy loss and/or shear stress.. From these calculations, a risk assessment for different scenarios of implantation parameters are given to the user. These different scenarios can include implanted medical device size, orientation, and implantation depth/height.

Example 3

Figure 3 shows an example of an in-vitro valve performance analysis. The user provides the artificial valve and information of the valve to the company. The 3D anatomy models to be tested are randomly selected from the database of patient-specific anatomies. Optical measurements are done in the selected physical model with the implanted valve. The fluid dynamics parameters from the blood flow in the selected conditions are calculated, giving the valve performance. A detailed report is sent to the user with the qualitative and quantitative information.

Example 4

Figure 4 illustrates the example of calculation of 4D MRI flow parameters in a patient- specific anatomy. The input is a stack of medical images from CT and/or 4D MRI. A trained mathematical model segments the images, which are used in the MRI data as a mask. The velocity vector field is created in the physical coordinate system in the masked image. The output is both qualitative and quantitative flow information including velocity, retrograde flow rate, kinetic energy, vorticity, helicity, turbulent intensity, energy loss and shear stresses. The output is stored in the database, and also shared with the user through an interface. The user can analyze the flow 4D at different sections of the anatomy and can get the time-averaged, space averaged, and temporal-spatial fluid dynamics parameters.

Example 5

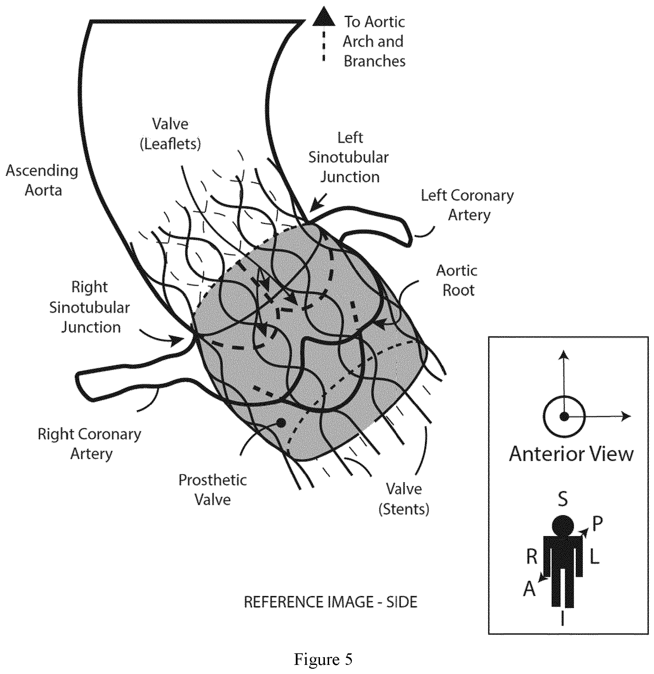

In the implantation procedure, the artificial heart valve is positioned on the diseased native valve. The prosthetic valve is positioned at the aortic root where the stents of the valve may extend towards the ascending aorta as shown in Figure 5. Depending on the size and the shape of the aortic valve, the implantation parameters may vary in patient specific anatomies.

Example 6

Valve design is one of the important parameters for the heart valve replacement operations. As depicted in Figure 6, the shape of the prosthetic valve may lead to different implantation positioning. Depending on the model, the stents, leaflets and the designs may vary and hence the positioning of the valves may vary in different anatomies.

Example 7

Orientation is one of the implantation parameters which needs to be considered during the operation. As shown in Figure 7, the orientation of the implanted valve may vary according to the position of the diseased native valve. The technology proposed allows to identify the optimum orientation, e.g., 45 degrees or 0 degrees. Physicians can select different orientations as an input to compare, and the algorithm allows to estimate post operational blood flow parameters for the selected valve orientations.

Example 8

Valve size is another important implantation parameter. It is possible to identify the optimum size before the operation using the proposed technology. Physicians can select different sizes as an input to compare and the algorithm allows to estimate post operational blood flow parameters for the selected valve sizes.

Example 9

Valve implantation depth is another crucial implantation parameter. It is possible to identify the optimum implantation depth before the operation using the proposed technology. Physicians can select different implantation depths as an input to compare and the algorithm allows to estimate post operational blood flow parameters for the selected implantation depths.

Example 10

Valve deployment thickness is another important implantation parameter which is possible to identify the optimum valve deployment thickness before the operation using the proposed technology. Physicians can select different valve deployment thicknesses as an input to compare and the algorithm allows to estimate post operational blood flow parameters for the selected valve deployment thicknesses.

Example 11

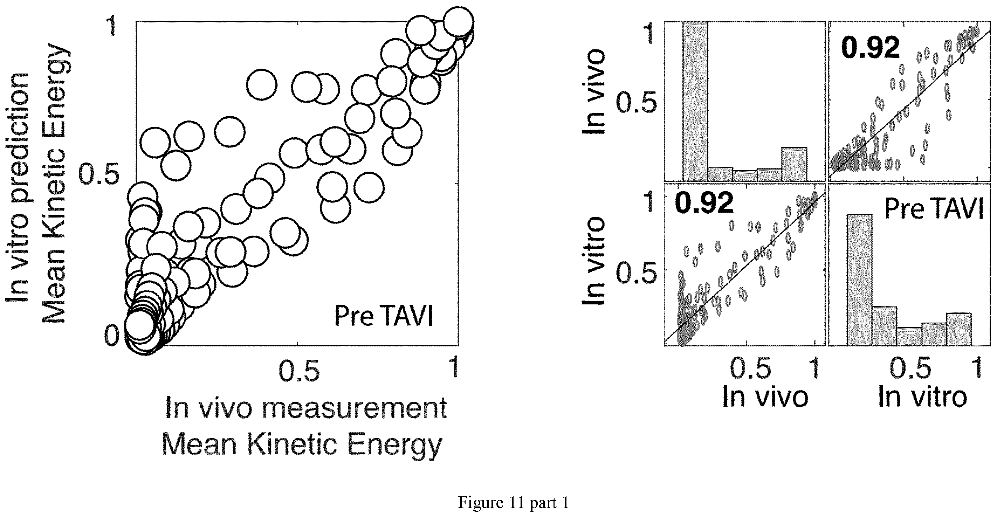

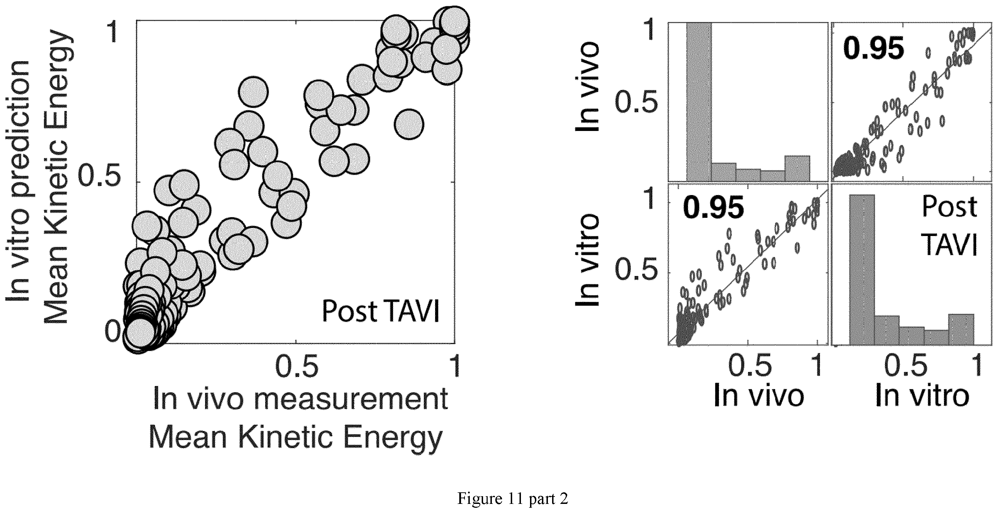

The preliminary results of the correlation between Mean Kinetic Energy (MKE) obtained via in vivo MRI measurement and the one obtained via invitro prediction for both before the actual TAVI implantation and after the TAVI operation is shown in Figure 11. Linear regression was utilized to define the correlation between in vivo measurements and in vitro predictions. Spatially averaged MKE values obtained via in vivo and in vitro for all phases of the heart cycle were compared. In total six patients were scanned before and after the TAVI operation. Based on their anatomical information, six different silicone models were 3D-printed. Finally in vitro optical measurements were performed using the anatomically accurate silicone models to extract the phase averaged blood velocity and MKE. The results show >92% agreemenmt for pre-TAVI and >95% agreement for post-TAVI cases.

**Claims**

1. A method for planning a heart valve implantation in a subject, the method comprising:

   (a) providing a subject-specific three-dimensional representation of the geometry of at least a portion of the heart and/or aorta and of blood flow therein,
   (b) training a mathematical model based on a training dataset comprising experimental blood flow characteristics in phantoms of at least the portion of the heart and/or aorta;
   (c) determining blood flow characteristics using the previously trained mathematical model in the presence and absence of an implanted heart valve based on the three-dimensional representation obtained in (a) and varied size, design, implantation depth, deployment thickness and/or orientation of the implanted heart valve, and
   (d) determining the size, design, implantation depth, deployment thickness and orientation of the implanted heart valve based on the determined blood flow characteristics in the presence and absence of the implanted heart valve,

   wherein the size, design, implantation depth, deployment thickness and/or orientation of the implanted heart valve are determined to minimize pressure drop, regurgitation volume, turbulence intensity, high shear stress regions, stagnation regions and/or flow separation regions.

2. The method according to claim 1, wherein the blood flow characteristics comprise blood flow patterns, velocity, retrograde flow rate, kinetic energy, vorticity, helicity, turbulence intensity, energy loss and/or shear stress.

3. The method according to claim 1 or 2, wherein the blood flow characteristics are temporally and/or spatially resolved.

4. The method according to any one of claims 1 to 3, wherein the blood flow characteristics are determined in systolic and/or diastolic phase.

5. The method according to any one of claims 1 to 4, wherein the previously trained mathematical model is a machine learning-based method, preferably a deep learning network.

6. The method according to any one of claims 1 to 5, wherein the three-dimensional representation of the geometry of the subject's heart and/or aorta is obtained from MRI and/or CT imaging, preferably wherein the MRI and/or CT images are automatically quantified and segmented by using a machine learning-based method, preferably wherein the machine learning-based method comprises a computer vision method.

7. The method according to any one of claims 1 to 6, wherein the previously trained mathematical model relies on a training dataset comprising experimental blood flow characteristics in phantoms of at least the portion of the heart and/or aorta.

8. The method according to claim 7, wherein the training dataset comprises the blood flow characteristics obtained from one or more subject(s) and/or through simulation(s).

9. The method according to any one of claims 1 to 8, wherein the phantom of at least the portion of the heart and/or aorta is a three-dimensional model, preferably obtained using additive manufacturing techniques according to the geometry of the heart and/or aorta of a subject, preferably using elastic and/or transparent material.

10. The method according to any one of claims 1 to 9, wherein the experimental blood flow characteristics are obtained using optical imaging techniques, preferably 2D/3D Particle Imaging and/or Particle Tracking Velocimetry.

11. The method according to any one of claims 1 to 10, wherein the training dataset comprises the experimental blood flow characteristics in the phantoms of at least the portion of the heart and/or aorta, determined in the presence of pathological conditions and/or implanted medical devices.


**Patentansprüche**

1. Verfahren zum Planen einer Herzklappenimplantation in einem Individuum, wobei das Verfahren umfasst:

(a) Bereitstellen einer Individuum-spezifischen dreidimensionalen Darstellung der Geometrie mindestens eines Teils des Herzens und/oder der Aorta und des Blutflusses darin,
(b) Trainieren eines mathematischen Modells auf der Grundlage eines Trainingsdatensatzes, der experimentelle Blutflusseigenschaften in anatomischen Modellen (phantoms) von mindestens dem Teil des Herzens und/oder der Aorta umfasst;
(c) Bestimmen der Blutflusseigenschaften unter Verwendung des zuvor trainierten mathematischen Modells in Anwesenheit und Abwesenheit einer implantierten Herzklappe auf Grundlage der in (a) erhaltenen dreidimensionalen Darstellung und variierter/n Größe, Designs, Implantationstiefe, Einsetzdicke (deployment thickness) und/oder Ausrichtung der implantierten Herzklappe, und
(d) Bestimmen der Größe, des Designs, der Implantationstiefe, der Einsetzdicke und der Ausrichtung der implantierten Herzklappe auf Grundlage der bestimmten Blutflusseigenschaften in Anwesenheit und Abwesenheit der implantierten Herzklappe,

wobei die Größe, das Design, die Implantationstiefe, die Einsetzdicke und/oder die Ausrichtung der implantierten Herzklappe bestimmt werden, um Druckabfall, Regurgitationsvolumen, Turbulenzintensität, Bereiche mit hoher Schubspannung (shear stress), Stagnationsbereiche und/oder Bereiche mit Strömungsablösung (flow separation regions) zu minimieren.

2. Verfahren nach Anspruch 1, wobei die Blutflusseigenschaften Blutflussmuster, Geschwindigkeit, retrograde Flussrate, kinetische Energie, Vortizität, Helizität, Turbulenzintensität, Energieverlust und/oder Schubspannung umfassen.

3. Verfahren nach Anspruch 1 oder 2, wobei die Blutflusseigenschaften zeitlich und/oder räumlich aufgelöst werden.

4. Verfahren nach einem der Ansprüche 1 bis 3, wobei die Blutflusseigenschaften in der systolischen und/oder diastolischen Phase bestimmt werden.

5. Verfahren nach einem der Ansprüche 1 bis 4, wobei das zuvor trainierte mathematische Modell ein auf Machine Learning beruhendes Verfahren ist, bevorzugt ein Deep-Learning-Netzwerk.

6. Verfahren nach einem der Ansprüche 1 bis 5, wobei die dreidimensionale Darstellung der Geometrie des Herzens und/oder der Aorta des Individuums aus MRT- und/oder CT-Bildern erhalten wird, bevorzugt wobei die MRT- und/oder CT-Bilder automatisch quantifiziert und segmentiert werden, indem ein auf Machine Learning beruhendes Verfahren verwendet wird, bevorzugt wobei das auf Machine Learning beruhende Verfahren ein Computer Vision-Verfahren umfasst.

7. Verfahren nach einem der Ansprüche 1 bis 6, wobei das zuvor trainierte mathematische Modell auf einem Trainingsdatensatz beruht, der experimentelle Blutflusseigenschaften in anatomischen Modellen von mindestens dem Teil des Herzens und/oder der Aorta umfasst.

8. Verfahren nach Anspruch 7, wobei der Trainingsdatensatz die Blutflusseigenschaften umfasst, die von einem oder mehreren Individuen und/oder durch Simulation(en) erhalten wurden.

9. Verfahren nach einem der Ansprüche 1 bis 8, wobei das anatomische Modell mindestens des Teils des Herzens und/oder der Aorta ein dreidimensionales Modell ist, das bevorzugt unter Verwendung additiver Fertigungstechniken entsprechend der Geometrie des Herzens und/oder der Aorta eines Individuums erhalten wird, bevorzugt unter Verwendung von elastischem und/oder transparenten Material.

10. Verfahren nach einem der Ansprüche 1 bis 9, wobei die experimentellen Blutflusseigenschaften unter Verwendung von optischen Bildgebungsverfahren, bevorzugt 2D-/3D-Particle Imaging und/oder -Particle Tracking Velocimetry, erhalten werden.

11. Verfahren nach einem der Ansprüche 1 bis 10, wobei der Trainingsdatensatz die experimentellen Blutflusseigenschaften in den anatomischen Modellen von mindestens dem Teil des Herzens und/oder der Aorta umfasst, die in Anwesenheit von pathologischen Zuständen und/oder implantierten medizinischen Vorrichtungen bestimmt werden.

**Revendications**

1. Procédé pour planifier l'implantation d'une valve cardiaque chez un sujet, le procédé comprenant les étapes suivantes :

    (a) fournir une représentation tridimensionnelle spécifique au sujet de la géométrie d'au moins une partie du cœur et/ou de l'aorte et de l'écoulement sanguin dans ceux-ci,
    (b) entraîner un modèle mathématique basé sur un ensemble de données d'entraînement comprenant des caractéristiques expérimentales du flux sanguin dans des fantômes d'au moins la partie du cœur et/ou de l'aorte ;
    (c) déterminer les caractéristiques du flux sanguin à l'aide du modèle mathématique préalablement formé en présence et en l'absence d'une valve cardiaque implantée sur la base de la représentation tridimensionnelle obtenue en (a) et de la taille, de la conception, de la profondeur d'implantation, de l'épaisseur de déploiement et/ou de l'orientation variables de la valve cardiaque implantée, et
    (d) déterminer la taille, la conception, la profondeur d'implantation, l'épaisseur de déploiement et l'orientation de la valve cardiaque implantée sur la base des caractéristiques de flux sanguin déterminées en présence et en l'absence de la valve cardiaque implantée, où la taille, la conception, la profondeur d'implantation, l'épaisseur de déploiement et/ou l'orientation de la valve cardiaque implantée sont déterminées afin de minimiser la chute de pression, le volume de régurgitation, l'intensité de la turbulence, les zones de contrainte de cisaillement élevée, les zones de stagnation et/ou les zones de séparation du flux.

2. Procédé selon la revendication 1, dans lequel les caractéristiques du flux sanguin comprennent des configurations de flux sanguin, la vitesse, le débit rétrograde, l'énergie cinétique, la vorticité, l'hélicité, l'intensité de la turbulence, la perte d'énergie et/ou la contrainte de cisaillement.

**3.** Procédé selon la revendication 1 ou la revendication 2, dans lequel les caractéristiques du flux sanguin sont résolues dans le temps et/ou dans l'espace.

**4.** Procédé selon l'une quelconque des revendications 1 à 3, dans lequel les caractéristiques du flux sanguin sont déterminées en phase systolique et/ou diastolique.

**5.** Procédé selon l'une quelconque des revendications 1 à 4, dans lequel le modèle mathématique préalablement entraîné est un procédé basé sur l'apprentissage automatique, de préférence un réseau d'apprentissage profond.

**6.** Procédé selon l'une quelconque des revendications 1 à 5, dans lequel la représentation tridimensionnelle de la géométrie du cœur et/ou de l'aorte du sujet est obtenue à partir d'une imagerie par IRM et/ou par tomodensitométrie, de préférence dans lequel les images IRM et/ou tomodensitométriques sont automatiquement quantifiées et segmentées à l'aide d'un procédé basé sur l'apprentissage automatique, de préférence dans lequel le procédé basé sur l'apprentissage automatique comprend un procédé de vision par ordinateur.

**7.** Procédé selon l'une quelconque des revendications 1 à 6, dans lequel le modèle mathématique préalablement entraîné repose sur un ensemble de données d'entraînement comprenant des caractéristiques expérimentales de flux sanguin dans des fantômes d'au moins la partie du cœur et/ou de l'aorte.

**8.** Procédé selon la revendication 7, dans lequel l'ensemble de données d'entraînement comprend les caractéristiques de flux sanguin obtenues à partir d'un ou plusieurs sujets et/ou par une ou plusieurs simulations.

**9.** Procédé selon l'une quelconque des revendications 1 à 8, dans lequel le fantôme d'au moins la partie du cœur et/ou de l'aorte est un modèle tridimensionnel, de préférence obtenu à l'aide de techniques de fabrication additive selon la géométrie du cœur et/ou de l'aorte d'un sujet, de préférence à l'aide d'un matériau élastique et/ou transparent.

**10.** Procédé selon l'une quelconque des revendications 1 à 9, dans lequel les caractéristiques expérimentales du flux sanguin sont obtenues à l'aide de techniques d'imagerie optique, de préférence par imagerie de particules 2D/3D et/ou par vélocimétrie par suivi de particules.

**11.** Procédé selon l'une quelconque des revendications 1 à 10, dans lequel l'ensemble de données d'entraînement comprend les caractéristiques expérimentales du flux sanguin dans les fantômes d'au moins la partie du cœur et/ou de l'aorte, déterminées en présence de conditions pathologiques et/ou de dispositifs médicaux implantés.

# Figures

Figure 1 part 1

Figure 1 part 2

Figure 2 part 1

Figure 2 part 2

```
┌─────────────────┐     ┌──────────────┐     ┌──────────────────┐     ┌────────────────┐     ┌─────────────┐
│ Valve information│     │ Database of 3D│     │ Optical flow      │     │ Fluid dynamics │     │ Valve       │
│(mechanical,      │ ──> │ printed models│ ──> │ measurements using│ ──> │ analysis       │ ──> │ performance │
│ physical)        │     │              │     │ anatomically      │     │ (in vitro flow │     │             │
└─────────────────┘     └──────────────┘     │ accurate          │     │ measurements)  │     └─────────────┘
                                              │ silicone model    │     └────────────────┘
                                              │ with implanted    │
                                              │ heart valve       │
                                              └──────────────────┘
```

```
              ┌──────────────────────────────────────────────────────────┐
              │                                                          │
┌──────────┐  │   ┌──────────┐              ┌──────────┐                │
│   User   │ ─┼─> │ receiving│ ───────────> │ database I│                │
│ interface│  │   │  unit I  │              └──────────┘                │
└──────────┘  │   └──────────┘                   │                      │
     ▲        │                                  ▼                      │
     │        │   ┌──────────┐              ┌──────────┐                │
     └────────┼── │ modelling│ <─────────── │ optical  │                │
              │   │   unit   │              │ imaging  │                │
              │   └──────────┘              └──────────┘                │
              │                                                          │
              └──────────────────────────────────────────────────────────┘
```

Figure 3

Figure 4



REFERENCE IMAGE - SIDE

Anterior View

Figure 5

VALVE 1 - SIDE

Left Sinotubular Junction

Left Coronary Artery

Ascending Aorta

Aortic Sinus

Right Sinotubular Junction

Aortic Root

Right Coronary Artery

Protshetic Valve

Aortic Annulus

Figure 6 part 1

VALVE 3 - SIDE

VALVE 2 - SIDE

Figure 6 part 2

Figure 7 part 1

Orientations - SIDE

0°

15°

Figure 7 part 2

EP 4 208 116 B1

Figure 8

EP 4 208 116 B1

Figure 9

Figure 10 part 1

Figure 10 part 2

Figure 11 part 1

Figure 11 part 2

EP 4 208 116 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015017571 A1
- US 2018174068 A

- US 20150112659 A

### Non-patent literature cited in the description

- **LAING JUSTIN et al.** *Journal of Medical Imaging, Society of Photo-Optical Instrumentation Engineers*, 01 April 2018, vol. 5 (2), 21222
- **RONNEBERGER, OLAF** ; **FISCHER, PHILIPP** ; **BROX, THOMAS**. U-Net: Convolutional Networks for Biomedical Image Segmentation. *arXiv:1505.04597*, 2015
- **GÜLAN, U. et al.** Investigation of atrial vortices using a novel right heart model and possible implications for atrial thrombus formation. *Scientifc Reports*, 2017, vol. 7, 16772, https://doi.org/10.1038/s41598-017-17117-3

- **GÜLAN, U.** ; **SAGUNER, A.M.** ; **AKDIS, D.** ; **GOTSCHY, A.** ; **TANNER, F.C.** ; **KOZERKE, S.** ; **MANKA, R.** ; **BRUNCKHORST, C.** ; **HOLZNER, M.** ; **DURU, F**. Hemodynamic Changes in the Right Ventricle Induced by Variations of Cardiac Output: A Possible Mechanism for Arrhythmia Occurrence. *Outflow Tract. Sci. Rep.*, 2019, vol. 9, 100
- **GÜLAN, U. et al.** Experimental study of aortic flow in the ascending aorta via particle tracking velocimetry. *Experiments in Fluids*, 2012, vol. 53, 1469-1485